# EUROPEAN PATENT APPLICATION

(11) **EP 1 398 039 A1**
(43) Date of publication of application: **17.03.2004**
(21) Application number: 03023804.2
(22) Date of filing: 21.01.1999
(51) Int. Cl.: A61K 38/18, A61K 48/00

(54) **Morphogenic proteins for enhancing cognitive function**

(30) Priority: 23.01.1998 US 12846
(62) Divisional of application: 99903241.0
(71) Applicant: Curis, Inc., Cambridge, MA 02138 (US)
(72) Inventor: Charette, Marc F., Needham MA 02192 (US)
(74) Representative: MacLean, Martin Robert

(57) **Abstract**

Disclosed are methods and compositions for protecting cognitive function in a mammal, particularly a human, subject to brain tissue damage, by administering a morphogen or a nucleic acid encoding a morphogen to the mammal. The methods and compositions can be used to reduce memory dysfunction and/or to provide a neuroprotective effect in subjects at risk of memory dysfunction resulting from a mechanical or chemical trauma, neuropathies, neurodegenerative diseases, or oxygen or glucose deprivation.

## Description

### Field of the Invention

The invention disclosed herein relates to use of morphogenic proteins in manufacture of medicaments for protecting, restoring, repairing, improving and/or correcting cognitive function in mammals.

### Background of the Invention

A class of proteins now has been identified that is competent to act as true tissue morphogens. That is, these proteins are able, on their own, to induce the migration, proliferation and differentiation of progenitor cells into functional replacement tissue. This class of proteins, referred to herein as "osteogenic proteins" or "morphogenic proteins" or "morphogens," includes members of the family of bone morphogenetic proteins (BMPs) identified by their ability to induce ectopic, endochondral bone morphogenesis. The morphogenic proteins generally are classified in the art as a subgroup of the TGF-β superfamily of growth factors (Hogan (1996) Genes & Development 10:1580-1594). Members of the morphogen family of proteins include the mammalian osteogenic protein-1 (OP-1, also known as BMP-7, and the *Drosophila* homolog 60A), osteogenic protein-2 (OP-2, also known as BMP-8), osteogenic protein-3 (OP-3), BMP-2 (also known as BMP-2A or CBMP-2A, and the *Drosophila* homolog DPP), BMP-3, BMP-4 (also known as BMP-2B or CBMP-2B), BMP-5, BMP-6 and its murine homolog Vgr-1, BMP-9, BMP-10, BMP-11, BMP-12, GDF3 (also known as Vgr2), GDF8, GDF9, GDF10, GDF11, GDF12, BMP-13, BMP-14, BMP-15, GDF-5 (also known as CDMP-1 or MP52), GDF-6 (also known as CDMP-2), GDF-7 (also known as CDMP-3), the *Xenopus* homolog Vgl and NODAL, UNIVIN, SCREW, ADMP, and NEURAL.

Members of this family encode secreted polypeptide chains sharing common structural features, including processing from a precursor "pro-form" to yield a mature polypeptide chain competent to dimerize and containing a carboxy terminal active domain, typically in the range of 97-106 amino acids. All members share a conserved pattern of cysteines in this domain and the active form of these proteins can be either a disulfide-bonded homodimer of a single family member or a heterodimer of two different members (see, *e*.*g*., Massague (1990) Annu. Rev. Cell Biol. 6:597; Sampath, et al. (1990) J. Biol. Chem. 265:13198). See also, U.S. 5,011,691; U.S. 5,266,683, Ozkaynak et al. (1990) EMBO J. 9: 2085-2093, Wharton et al. (1991) PNAS 88:9214-9218), (Ozkaynak (1992) J. Biol. Chem. 267:25220-25227 and U.S. 5,266,683); (Celeste et al. (1991) PNAS 87:9843-9847); (Lyons et al. (1989) PNAS 86:4554-4558). These disclosures describe the amino acid and DNA sequences, as well as the chemical and physical characteristics, of these osteogenic proteins. See also, Wozney et al. (1988) Science 242:1528-1534); BMP 9 (W093/00432, published January 7, 1993); DPP (Padgett et al. (1987) Nature 325:81-84; and Vg-1 (Weeks (1987) Cell 51:861-867).

The morphogenic activities of these proteins allow them to initiate and maintain the developmental cascade of tissue morphogenesis in an appropriate, morphogenically permissive environment, stimulating stem cells to proliferate and differentiate in a tissue-specific manner, and inducing the progression of events that culminate in new tissue formation. These morphogenic activities also allow the proteins to stimulate the "redifferentiation" of cells previously induced to stray from their differentiation path. The proteins are useful in the replacement of diseased or damaged tissue in a mammal, particularly when the damaged tissue interferes with normal tissue or organ function, such as, for example, damaged lung tissue resulting from emphysema; cirrhotic kidney or liver tissues; damaged heart or blood vessel tissue, as may result from cardiomyopathies and/or atherothrombotic or cardioembolic strokes; damaged stomach tissue resulting from ulceric perforations or their repair; damaged neural tissue, as may result from physical injury, degenerative diseases such as Alzheimer's disease or multiple sclerosis or strokes; damaged dentin and periodontal tissues, as may result from disease or mechanical injury.

The proteins have been shown to have utility in protecting, restoring, repairing and/or regenerating a number of non-chondrogenic tissues, including dentin, liver, kidney, neural, cardiac lung, gastrointestinal tract tissue and the like. See, for example, W902/15323, published September 17, 1992; W093/04692, published March 18, 1993; W094/06399, published March 31, 1994; W094/03200, published February 17, 1994; W094/06449, published March 31, 1993; W094/06420, published March 31, 1994. See also, USSN 08/404,113; 08/445,467; 08/432,883; 08/155,343; 08/260,675; 08/445,468; 08/461,397; 08/480,528; 08/402,542; 08/396,930; 08/751,227; 08/620,444, and 08/926,154, the disclosures of which are incorporated herein by reference.

Brain tissue has little or no ability to regenerate following tissue damage. Nor does the brain store oxygen or glucose. Thus, brain tissue damage as a result of cell loss, tissue trauma, oxygen or glucose deprivation, or cell or tissue degeneration, can result in permanent brain damage. One primary consequence of cerebral tissue damage or cell loss, is impaired cognitive function, particularly impaired memory. Transient or permanent memory disorders are a primary and devastating symptom of strokes, myocardial infarctions (heart attacks) and other ischemic traumas to the body. As one example, impaired cognitive function, including memory deficits and difficulty in learning, are found in up to 20% of patients surviving severe heart attacks and constitute one of the most frequent and enduring disabilities associated with such ischemic events. Memory disorders also are a primary symptom of a number of neurodegenerative disorders, including Alzheimer disease, Pick disease and Lewy-body disease. Dementia also is a prevalent symptom in Huntington's chorea, Parkinson's disease, ALS, dementia pugilista and various other disorders characterized by cerebral atrophy. Memory dysfunction also results from the cell loss and/or cell senescence associated with aging and which can lead to senility.

Needs remain for therapies for enhancing, improving, repairing, restoring and/or protecting cognitive function in a mammal, particularly a human at risk for, or suffering from, a memory deficit or disorder or dysfunction. To date, a promising means for repairing loss of cognitive function has not been identified.

Accordingly, it is an object of the instant invention to provide uses of morphogens in manufacture of medicaments for enhancing, improving, repairing, restoring and/or protecting cognitive function in a mammal, particularly a human at risk for, or suffering from, a memory deficit, disorder or dysfunction. It is another object of the invention to provide uses of proteins, and/or nucleic acids encoding these proteins, in manufacture of medicaments that can be administered to the body prior to, or following, tissue injury. These and other objects, along with advantages and features of the invention disclosed herein, will be apparent from the description, drawings and claims that follow.

### Summary of the Invention

It now has been discovered that morphogenic proteins ("morphogens") can be used to protect and/or improve or repair cognitive function in a mammal, particularly a human. The discovery has particular application in contexts where injury to brain tissue has affected or otherwise impaired the individual's cognitive function. The uses enabled by this disclosure can reduce memory dysfunction in a manunal, including memory deficits, amnesia and/or impaired spatial or non-spatial stimulus-stimulus associations. The morphogenic protein itself can be administered directly to the individual, or a nucleic acid encoding the morphogen can be administered. Furthermore, in view of the existing knowledge of cognitive function and cerebral tissue in animals, the instant discovery is unexpected and contravenes the art's current understanding of cognitive function therapies. In one aspect, the uses of the invention can protect cognitive function in a mammal at risk of, or suffering from, brain tissue damage associated with oxygen or glucose deprivation to the brain. Sources of such damages include ischemia and malnutrition. In one embodiment the individual is at risk of, or suffers from, a stroke, as can result from arteriosclerosis (coronary artery occlusion), an embolism, an aneurysm, cardiac arrest (heart attack) or multiple cardiac infarcts. In another embodiment, ischemia occurs as part of a surgical procedure, particularly one involving blood loss. In still another embodiment, the individual is at risk of, or suffers from, malnutrition, particularly severe or chronic malnutrition, as can result from glucose metabolism disorders, including diabetes, and/or lack of food, as can result from starvation, anorexia and/or bulimia.

In another aspect, the uses of the invention can protect cognitive function in a mammal at risk of, or suffering, from brain tissue damage associated with trauma, including mechanical or chemical trauma. In one embodiment, mechanical trauma can follow a concussion and other blows to the head, as well as other forms of mechanical trauma to the body resulting in cognitive dysfunctions or disorders. In another embodiment, chemical traumas include neurotoxins, including lead, ethanol and other alcohols, ammonia, formaldehyde and many other organic solvents, as well as toxins, cigarette smoke and opiates.

In another aspect, the uses can treat and/or reduce memory dysfunction associated with hippocampal tissue damage and/or medial temporal lobe tissue damage. In one embodiment, the uses can treat an individual with hippocampal CA1 and/or dentate hilar neuronal damage and alleviate symptoms associated with this damage.

In still another aspect, the uses can treat and/or alleviate a symptom of a neurodegenerative disorder, including disorders characterized by cerebral atrophy, senility and/or dementia. Several known types of these disorders include Alzheimer disease, Pick disease, Lewy-body disease, Huntington's chorea, the dementia associated with Parkinson's disease, amylotrophic lateral sclerosis, cortical-basal ganglionic degeneration, polyglucosa body disease, dementia pugilista, cortical-stnatal-spinal degeneration, and thalamic dementia.

In still another aspect the invention provides uses of morphogens for enhancing, protecting, repairing or restoring cognitive function affected by neuronal cell loss, including cell death or cell senescence. In one embodiment the cell loss is a phenomenon of aging. Thus, in one aspect, the invention provides uses for protecting, repairing, restoring, enhancing or ameliorating memory function in an aging individual, including prior to, or after the onset of senility.

In still another aspect, the invention provides use of a morphogens or a nucleic acid encoding the morphogen in manufacture of a medicament for administering to an individual to protect restore, repair or enhance cognitive function in the mammal. The protein or nucleic acid preferably can be provided directly, as by injection or by a passive pump provided intraperitoneally, intraventricularly, intravenously or intracisternally. Alternatively, cells competent to express and secrete the protein can be implanted in the individual.

In another aspect, the instant invention provides a kit for practice of the above-described methods. As contemplated herein, one embodiment of a kit for reducing cognitive function loss and/or enhancing cognitive function comprises protein or nucleic acid and a liquid carrier packaged in the same receptacle. In other embodiments, the morphogenic protein or nucleic acid is provided in lyophilized form and reconstituted in an aqueous buffer in the same receptacle, which can include a syringe. In still another embodiment, cells competent to express the protein are provided together with any hollow fiber membrane. The cells can be provided in the fabricated device together with morphogen in a liquid carrier. Alternatively the kit can comprise cells and the membrane as separate components to be fabricated just prior to use.

In one preferred embodiment, the protein is provided as a liquid formulation administered intravenously. In another embodiment, the protein is provided in a liquid formulation intraperitoneally. In still another embodiment, the protein is provided in a form for intracisternal or intraventricular administration. In all these administration forms, the protein or nucleic acid can be administered "naked", associated with a biocompatible excipient, or disposed in biocompatible, biodegradable or bioerodible microspheres and other delivery vehicles, including matrices competent to allow sustained release of the molecule over time, or otherwise combined with suitable binding agents as described herein. Another preferred embodiment can have a dry powder configuration that is solubilized just prior to administration. A suitable formulation results from first dispersing morphogenic protein in a liquid carrier such as water with or without excipient, followed by lyophilization. In one formulation the composition is a solution made by combining the protein together with an acidic buffered solution, e.g., pH 4.0-4.5, for example an acetate or citrate buffer. Still another formulation is a suspension formed by disbursing morphogenic protein in a physiologically buffered solution, such as phosphate buffered saline (PBS). In still another embodiment morphogen-producing cells can be implanted in an individual. In one embodiment, the cells can be part of a device that contains the cells and allows the protein to be secreted from the device into the surrounding cerebral tissue. In another embodiment, the cells are implanted directly.

As described above, the invention provides, in one aspect, novel means for protecting, restoring, repairing, or enhancing cognitive function by administering morphogenic protein to the afflicted individual. As contemplated herein, useful morphogens include morphogenic proteins such as, but not limited to, OP-1, OP-2, BMP-2, BMP-4, BMP-5 and BMP-6. A currently preferred morphogenic protein is OP-1. As used herein, the terms "morphogen", "bone morphogen", "bone morphogenic protein", "BMP", "osteogenic protein" and "osteogenic factor" embrace the class of proteins typified by human osteogenic protein 1 (hOP-1). Nucleotide and amino acid sequences for hOP-1 are provided in Seq. ID Nos. 1 and 2, respectively. For ease of description, hOP-1 is recited herein below as a representative morphogenic protein. It will be appreciated by the artisan of ordinary skill in the art, however, that OP-1 merely is representative of the TGF-β subclass of true tissue morphogens, and is not intended to limit the description. Other known, and useful proteins include, BMP-2, BMP-3, BMP-3b, BMP-4, BMP-5, BMP-6, BMP-8, BMP-9, BMP-10, BMP-11, BMP-12, BMP-13, BMP-15, GDF-1, GDF-2, GDF-3, GDF-5, GDF-6, GDF-7, GDF-8, GDF-9, GDF-10, GDF-11, GDF-12, NODAL, UNIVIN, SCREW, ADMP, NEURAL and morphogenically active amino acid variants thereof. In one preferred embodiment, the proteins useful in the invention include biologically active species variants of any of these proteins, including conservative amino acid sequence variants, proteins encoded by degenerate nucleotide sequence variants, and osteogenically active proteins sharing the conserved seven cysteine skeleton as defined herein and encoded by a DNA sequence competent to hybridize to a DNA sequence encoding a morphogenic protein disclosed herein, including, without limitation, OP-1, BMP-5, BMP-6, BMP-2, BMP-4 or GDF-5, GDF-6 or GDF-7. In another embodiment, useful osteogenic proteins include those sharing the conserved seven cysteine domain and sharing at least 70% amino acid sequence homology (similarity) within the C-terminal active domain, as defined herein. In another embodiment, useful proteins include those sharing greater than 60% identity in the C-terminal domain. In still another embodiment, useful osteogenic proteins can be defined as osteogenically active proteins having any one of the generic sequences defined herein, including OPX (SEQ ID No: 3) and Generic Sequences 7 and 8 (Seq. ID Nos. 4 and 5), or Generic Sequences 9 and 10 (Seq. ID Nos. 6 and 7).

In any use of the invention, "administering morphogenic protein" refers to administering the protein, alone or in combination with other molecules, or administering a nucleic acid encoding the morphogen or a biologically active fragment thereof, and competent to be expressed *in vivo*. Useful nucleic acids include DNA or RNA. Useful forms of the protein include, for example, the mature form of the morphogen provided alone or provided in association with its precursor "pro" domain, which is known to enhance the solubility of the protein. As used herein, "soluble complex" of a morphogenic protein is understood to mean the dimeric species complexed with part or all of a morphogenic protein pro domain. See, for example, W094/03600, published 18 Feb. 1994 and/or Jones et al., (1994) Growth Factors 11:215-225, for a detailed description of the soluble complex form of morphogenic proteins. Other useful molecules known to enhance proton solubility include, without limitation, casein and other milk components, as well as various serum proteins.

Also contemplated within the scope of the invention is use of a morphogen analog, that is, any molecule competent to induce a morphogen-mediated biological effect in a mammal. Thus, as used herein, a morphogen analog is any substance that mimics morphogen activation of the regulatory pathway of phenotype-specific gene expression, inducing at least one "morphogen-mediated biological effect" in a cell or tissue. Examples include naturally-occurring or synthetic molecules that are peptide-based or non-peptide based analogs, including small molecule numetics, and antibodies, antibody fragments and single chain antibody binding sites ("BABS"). Useful molecules include naturally-occurring or synthetic morphogen homologs or "mimetics", that is, molecules that functionally substitute for a morphogen by binding to the same cell surface receptor binding sites and activating the receptor as a morphogen does. Other useful molecules include those that occur upstream or downstream of morphogen activation in the morphogen-activated regulatory pathway and homologs, analogs or mimetics of these upstream or downstream molecules, and/or molecules that modulate expression of upstream or downstream members of the pathway.

The foregoing and other objects, features and advantages of the present invention will be made more apparent from the following detailed description of the invention.

### Brief Description of the Drawings

The foregoing and other objects and features of this invention, as well as the invention itself, may be more fully understood from the following description, when read together with the accompanying drawings, in which like-referenced components are similarly numbered:
FIG. 1 presents the percent amino acid sequence identity and percent amino acid homology ("similarity") that various members of the family of morphogenic proteins as defined herein share with OP-1 in the C-terminal seven cysteine domain;
FIG. 2 is a schematic representation of a transitive pair-association assay described herein, and
FIG. 3 is a schematic representation of a symmetry pair association assay described herein.
FIG. 4 is a schematic representation of one morphogen-activated regulatory pathway for expression of a phenotype-specific gene.

### Detailed Description of Preferred Embodiments

It now has been discovered that motphogenic proteins are competent to protect, repair, restore, or enhance cognitive function in a mammal, particularly a human. The proteins, or nucleic acids encoding them, can be provided to an individual at risk of, or suffering from, cerebral tissue damage to alleviate symptoms of cognitive function loss associated with the injury. The brain tissue damage can be induced by trauma to the brain such as by mechanical or chemical trauma, by a neurotoxin, by oxygen or glucose deprivation to the brain, such as results from an interruption of blood flow to the brain, by tissue loss from a neurodegenerative disorder, or by any action resulting in brain cell loss or senescence, including aging. The proteins, or the nucleic acids encoding them, can be administered intravenously, intraventricularly, intraperitoneally, or by any other suitable means. As a result of the instant discovery therapeutic means for alleviating memory dysfunction in humans now are available.

Provided below are detailed descriptions of suitable morphogenic proteins and formulations suitable for the uses, of this invention, as well as methods for the administration and application of the resulting medicaments, and numerous, nonlimiting examples which 1) illustrate the suitability of the uses of morphogenic proteins described herein for protecting, restoring, repairing, improving and or correcting cognitive function in mammals; and 2) provide assays with which to test candidate proteins and formulations for their efficacy.

In order to more clearly and concisely describe the subject matter of the claimed invention, the following definitions are intended to provide guidance as to the meaning of specific terms used in the written description and appended claims.

As used herein, "brain tissue" and "cerebral tissue" are use interchangeably. "Disorder" "deficit" and "dysfunction" all are terms intended to describe a loss or reduction or malfunctioning of a specified function. As used herein, "cognitive function" is understood to mean "of or pertaining to the mental processes of perception, memory, judgment and reasoning" (Random House Dictionary, Unabridged, 2nd ed., Random House, NY 1987). See also, Taber's cyclopedic Medical Dictionary, F.A. Davison C.V., Philadelphia, 1989.

As embodied herein, the expression "maintaining normal tissue function" means both regaining or restoring tissue function e.g., cognitive function, lost due to an injury or acquired or congenital defect, as well as protecting the tissue at risk of damage from injury. Restoring tissue function can include regenerating new tissue and/or simulating existing differentiated tissue cells to continue expressing their phenotype as in the case of senescent cells. "Depressed tissue function" level refers to a diminished to deficient tissue function as a result of a tissue injury or disease. The expression "enhance viability of" a tissue or organ, as used herein, means protection from, reduction of and/or elimination of reduced or lost tissue or organ function as a result of tissue necrosis, senescence and/or fibrosis, particularly immune response-mediated tissue necrosis and/or fibrosis. "Alleviating" means protection from, reduction of and/or elimination of, undesired tissue dysfunction, or reducing or eliminating a symptom associated with the tissue dysfunction. Here the tissue dysfunction would be associated with a reduction in cognitive function. "Treating" means providing the protein or nucleic acid to an individual as part of a therapy designed to protect an individual from the tissue dysfunction and/or to provide an ameliorative or palliative effect to an individual afflicted with the tissue dysfunction. "Morphogenically permissive environment" is understood to mean an environment competent to allow tissue morphogenesis to occur. Finally, "symptom alleviating cofactor" refers to one or more pharmaceuticals which may be administered together with the therapeutic composition of this invention and which alleviate or mitigate one or more of the symptoms typically associated with the tissue injury and/or tissue function loss. Exemplary cofactors include antibiotics, antiseptics, non-steroidal anti-inflammatory agents, and the like.

"Defect" or "defect site" or "defect locus", as contemplated herein, can define any structural or functional disruption in a tissue or organ requiring repair or treatment.

"Repair" is intended to mean formation of new tissue which is sufficient to restore or improve function and/or otherwise functionally correct a defect in a mammal. Repair does not, however, mean, or otherwise necessitate, a process of complete healing or a treatment which is 100% effective at restoring a defect to its pre-defect physiological/structural/mechanical state.

In addition to morphogenic proteins, various systemic factors, hormones, enzymes, enzyme inhibitors and/or chemoattractant/chemotactic factors, therapeutic compositions, antibiotics, or other bioactive agents also can be contained within a formulation for use in the invention. Thus, various known growth factors such as EGF, PDGF, IGF, FGF, NGF, GDNF, NT-3, NT-4, TGF-α, and TGF-β can be combined with a morphogenic formulation described herein.

"Morphogen", "morphogenic protein", "osteogenic protein", or "bone morphogenic protein," generally is understood to mean a protein which can induce the full cascade of morphogenic events culminating in new organ-specified tissue formation. As described elsewhere herein, the class of proteins is typified by human osteogenic protein (hOP1). Other osteogenic proteins useful in the practice of the invention include osteogenically active forms of OP1, OP2, OP3, BMP2, BMP3, BMP4, BMP5, BMP6, BMP9, DPP, Vgl, Vgr, 60A protein, GDF-1, GDF-3, GDF-5, 6, 7, BMP10, BMP11, BMP12, BMP13, BMP15, UNIVIN, NODAL, SCREW, ADMP or NEURAL and amino acid sequence variants thereof. In one currently preferred embodiment, osteogenic protein includes any one of: OP1, OP2, OP3, BMP2, BMP4, BMP5, BMP6, BMP9, and amino acid sequence variants and homologs thereof, including species homologs thereof. Particularly preferred proteins are those comprising an amino acid sequence having at least 70% homology with the C-terminal 102-106 amino acids, defining the conserved seven cysteine domain, of human OP-1, BMP2, and related proteins. Certain preferred embodiments of the instant invention comprise the osteogenic protein, OP-1 and proteins sharing greater than 60% amino acid sequence identity with OP-1 in the C-terminal seven cysteine domain. Certain other preferred embodiments comprise mature OP-1 solubilized in a physiological saline solution. As further described elsewhere herein, the proteins suitable for use with the invention can be identified by means of routine experimentation using the art-recognized bioassay described by Reddi and Sampath. A detailed description of useful morphogenic proteins is provided below.

"Binding agent", as used herein, means any physiologically-compatible material which, when admixed with a morphogenic protein as defined herein, enhances a desired physical property of the formulation without substantially destroying the biological activity of the protein *in vivo*. Among the other characteristics of a preferred binding agent is an ability to keep the protein or nucleic acid localized at a site of interest and/or to render the device pliable, shapeable and/or malleable. Additionally, in certain preferred embodiments, a binding agent can achieve the aforementioned features and benefits when present in low proportions.

Those binding agents contemplated as useful herein include, but are not limited to: art recognized suspending agents, viscosity-producing agents and emulsifying agents. In particular, art-recognized agents, such as cellulose gum derivatives, sodium alginate, and gelatin powder can be used. More particularly, cellulosic agents such as alkylcelluloses, are preferred including agents such as methylcellulose, methythydroxyethylcellulose, hydroxyethylcellulose, hydroxypropylmethylcellulose, carboxymethylcellulose, sodium carboxymethylcellulose, and hydroxyalkylcelluloses, to name but a few. Currently, the most preferred is carboxymethylcellulose, including the sodium salt thereof. Other useful binding agents include, but are not limited to, dextran, mannitol, white petrolatum, sesame oil and admixtures thereof. Still other useful agents include materials commonly referred to in the art as "matrix" materials, including hydroxyapatite, calcium carbonates, bioactive ceramics, insoluble or solubilized collagen, synthetic polymers of glycolic acid, butyric acid and/or lactic acid monomers, and the like, including mixtures comprising any one or more of the foregoing materials. In view of the teachings set forth herein, the artisan can identify suitable equivalents of the above-identified binding agents using merely routine experimentation and ordinary skill.

"Wetting agent" or "carrier", as used herein, means any physiologically-compatible aqueous solution, provided it does not interfere with the *in vivo* biological activity of the osteogenic protein. Currently preferred wetting or carrier agents include aqueous solutions competent to solubilize or otherwise suspend the protein in solution such that it can be administered in liquid form to an individual. Currently preferred carriers include, without limitation, physiological saline, phosphate buffered saline (PBS), acetate buffered solutions (pH4.5) and the like. Equivalents can be identified by the artisan using no more than routine experimentation and ordinary skill.

Without being limited to any one theory, the morphogen can be used in the uses of the present invention upon injury to, tissue contemplated to result in loss of cognitive function, or in anticipation of such injury, for a time and at a concentration sufficient to prevent or reduce loss of cognitive function, and/or to restore lost function. Restoring function may include repairing damaged tissue -including cerebral tissue and/or neural pathways, or forming new tissue and/or inhibiting additional damage thereto.

The morphogens also are useful for enhancing survival of neuronal cells at risk of dying, thereby preventing, limiting or otherwise inhibiting damage to tissue and/or neural pathways which result in memory loss or dementia. Non-mitotic neurons are at risk of dying as a result of a neuropathy or other cellular dysfunction of a neuron or glial cell inducing cell death, or following a chemical or mechanical lesion to the cell or its surrounding tissue. The chemical lesions may result from known toxic agents, including lead, ethanol, animonia, formaldehyde and many other organic solvents, as well as the toxins in cigarette smoke and opiates. Excitatory amino acids, such as glutamate also may play a role in the pathogenesis of neuronal cell death (see Freese et al. (1990) Brain Res. 521:254-264). Neuronal cell death also is thought to be a significant contributing factor in a number of neurodegenerative diseases, including Alzheimer's disease, Huntington's chorea, Parkinson's disease, amyotrophic lateral sclerosis (ALS) and multiple sclerosis. The etiology of these neuropathies may be, infectious, toxic, autoimmune, nutritional, ischemic or metabolic, such as results in hepatic encephalopathy. In addition, ethanol and a number of other toxins also have been identified as significant contributing factors in neurodegenerative diseases.

The morphogens described herein also are useful for providing neuroprotective effects to alleviate neural pathway damage associated with the body's immune/inflammatory response to an initial injury to nerve tissue, which can result in cognitive function loss. Such a response may follow trauma to nerve tissue, caused, for example, by an autoimmune dysfunction, neoplastic lesion, infection, chemical or mechanical trauma, disease, by interruption of blood flow to the neurons or glial cells, for example following ischemia or hypoxia, or by other trauma to the nerve or surrounding material. For example, at least part of the damage resulting from hypoxia or ischemia-reperfusion following occlusion of a neural blood supply, as in an embolic stroke, is believed to be immunologically associated. In addition, at least part of the damage associated with a number of primary brain tumors also appears to be immunologically related. Providing morphogen to the mammal may be used to alleviate and/or inhibit the immunologically related response to a neural injury and associated with memory dysfunction or dementia. Where the injury is to be induced, as during surgery or other aggressive clinical treatment, the morphogen or agent may be provided prior to induction of the injury to provide a neuroprotective effect to the nerve tissue at risk.

In still another aspect, the morphogens described herein provide means for supporting the growth and maintenance of differentiated neurons, including inducing neurons to continue expressing their phenotype. It is anticipated that this activity will be particularly useful in the treatment of nerve tissue disorders where loss of function is caused by reduced or lost cellular metabolic function and cells become senescent or quiescent. This reduction in metabolic function is thought to occur in aging cells and to be manifested in Alzheimer's disease, and can result in amnesia, memory loss, senility and/or dementia. Providing morphogen can stimulate these cells to continue expressing their phenotype, significantly inhibiting and/or reversing the effects of the cellular metabolic dysfunction, thereby maintaining the neural pathway at risk.

The invention also can be used for treating amnesia associated with traumatic injuries to the central nervous system (CNS) that are caused by mechanical forces, such as a blow to the head. Trauma can involve a tissue insult such as results from abrasion, incision, contusion, puncture, compression, etc., such as can arise from traumatic contact of a foreign object with any locus of or appurtenant to the mammalian head, neck or vertebral column. Other forms of traumatic injury can arise from constriction or compression of mammalian CNS tissue by an inappropriate accumulation of fluid (*e*.*g*., a blockade or dysfunction of normal cerebrospinal fluid or vitreous humor fluid production, turnover or volume regulation, or a subdural or intracranial hematoma or edema). Similarly, traumatic constriction or compression can arise from the presence of a mass of abnormal tissue, such as a metastatic or primary tumor.

The means for making and using the medicaments of the invention, as well as other material aspects concerning their nature and utility, including how to make and how to use the subject matter claimed, will be further understood from the following, which constitutes the best mode currently contemplated for practicing the invention. It will be appreciated that the invention is not limited to such exemplary work or to the specific details set forth in these examples.

### I. MORPHOGEN CONSIDERATIONS

### A. Biochemical, Structural and Functional Properties of Bone Morphogenic Proteins

In its mature, native form, natural-sourced morphogenic protein is a glycosylated dimer typically having an apparent molecular weight of about 30-36 kDa as determined by SDS-PAGE. When reduced, the 30 kDa protein gives rise to two glycosylated peptide subunits having apparent molecular weights of about 16 kDa and 18 kDa. In the reduced state, the protein has no detectable osteogenic activity. The unglycosylated protein, which also has osteogenic activity, has an apparent molecular weight of about 27 kDa. When reduced, the 27 kDa protein gives rise to two unglycosylated polypeptide chains, having molecular weights of about 14 kDa to 16 kDa. Typically, the naturally occurring osteogenic proteins are translated as a precursor, having an N-terminal signal peptide sequence typically less than about 30 residues, followed by a "pro" domain that is cleaved to yield the mature C-terminal domain. The signal peptide is cleaved rapidly upon translation, at a cleavage site that can be predicted in a given sequence using the method of Von Heijne (1986) Nucleic Acids Research 14:4683-4691.

Morphogens comprise a pair of polypeptide chains that, when folded, adopt a configuration sufficient for the resulting dimeric protein to elicit morphogenetic responses in cells and tissue displaying receptors specific for said morphogen. That is, morphogens generally induce all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells. "Progenitor" cells are uncommitted cells that are competent to differentiate into one or more specific types of differentiated cells, depending on their genomic repertoire and the tissue specificity of the permissive environment in which morphogenesis is induced. Morphogens further can delay or mitigate the onset of senescence- or quiescence-associated loss of phenotype and/or tissue function. Morphogens still further can stimulate phenotypic expression of differentiated cells, including expression of metabolic and/or functional, *e*.*g*., secretory, properties thereof. In addition, morphogens can induce redifferentiation of committed cells under appropriate environmental conditions. As noted above, a morphogen that induces proliferation and/or differentiation of at least one of the following tissues: dentin, cardiac, lung, liver, renal, adrenal, thyroid, ovarian, spleen, neural, pancreas, or gastrointestinal tract tissue, and/or support the growth, maintenance and/or functional properties of any of these tissues, is of particular interest herein.

Morphogenic proteins useful herein include any known naturally-occurring native proteins including allelic, phylogenetic counterpart and other variants thereof, whether naturally-occurring or biosynthetically produced (*e*.*g*., including "muteins" or "mutant proteins"), as well as new, biologically active members of the general morphogenic family of proteins.

Particularly useful sequences include those comprising the C-terminal 96 or 102 amino acid sequences ofDPP (from Drosophila), Vgl (from Xenopus), Vgr-1 (from mouse), the OP1 and OP2 proteins, proteins (see U.S. Pat. No. 5,011,691 and Oppermann *et al*., as well as the proteins referred to as BMP2, BMP3, BMP4 (see W088/00205, U. S. Patent No. 5,013,649 and W091/18098), BMP5 and BMP6 (see W090/11366, PCT/US90/01630), BMP8 and BMP9. Other proteins useful in the practice of the invention include active forms of OP1 OP2, OP3, BMP2, BMP3, BMP4, BMP5, BMP6, BMP9, GDF-5, GDF-6, GDF-7, DPP, Vgl, Vgr, 60A protein, GDF-1, GDF-3, GDF-5, GDF-6, GDF-7, BMP10, BMP11, BMP13, BMP15, UNIVIN, NODAL, SCREW, ADMP or NURAL and amino acid sequence variants thereof. In one currently preferred embodiment, morphogenic protein include any one of: OP 1, OP2, OP3, BMP2, BMP4, BMP5, BMP6, BMP9, and amino acid sequence variants and homologs thereof, including species homologs, thereof.

Publications disclosing these sequences, as well as their chemical and physical properties, include: OP-1 and OP-2: U.S. 5,011,691, U.S. 5,266,683, Ozkaynak *et al. (1990) EMBO J 9:* 2085-2093; OP-3: W094/10203 (PCT US93/10520); BMP2, BMP3, BMP4: W088/00205, Wozney *et al*. (*1988*) *Science 242:* 1528-1534); BMP5 and BMP6: Celeste *et al (1991) PNAS* 87: 9843-9847; Vgr-1: Lyons *et al (1989) PNAS 86:* 4554-4558; DPP: Padgett *et al*. (1987) *Nature 325:* 81-84; Vg-1: Weeks (1987) *Cell 51:* 861-867; BMP-9: W095/33830 (PCT/US95/07084); BMP10: W094/26893 (PCT/LTS94/05290); BMP-11: W094/26892 (PCT/US94/05288); BMP12: W095/16035 (PCT/US94/14030); BMP-13: W095/16035 (PCT/US94/14030); GDF-1: W092/00382 (PCT/US91/04096) and Lee *et al. (1991) PNAS 88:* 4250-4254; GDF-8: W094/21681 (PCT/US94/03019); GDF-9: W094/15966 (PCT/US94/00685); GDF-10: W095/10539 (PCT/US94/11440); GDF-11: W096/01845 (PCT/US95/08543); BMP-15: W096/36710 (PCT/US96/06540); MP121: W096/01316 (PCT/EP95/02552); GDF-5 (CDMP-1, MP52): W094/15949 (PCT/US94/00657) and W096/14335 (PCT/US94/12814) and W093/16099 (PCT/EP93/00350) and Stone *et al*., (1994), *Nature* 368:639-643; GDF-6 (CDMP-2, BMP 13): W095/01801 (PCT/US94/07762) and W096/14335 and W095/10635 (PCT/US94/14030); GDF-7 (CDMP-3, BMP12): W095/10802 (PCT/US94/07799) and W095/10635 (PCT/US94/14030); BMP-3b: Takao, et al., (1996), *Biochem. Biophys. Res. Comm*. 219:656-662; GDF-3: W094/15965; 60A: Blaster et al., (1993), Cell 73:687-702 and GenBank accession number L12032. In another embodiment, useful proteins include biologically active biosynthetic constructs, including novel biosynthetic proteins and chimeric proteins designed using sequences from two or more known osteogenic proteins. See also the biosynthetic constructs disclosed in U.S. Pat. 5,011,691, the disclosure of which is incorporated herein by reference (*e*.*g*., COP-1, COP-3, COP-4, COP-5, COP-7, and COP-16).

In certain preferred embodiments, morphogenic proteins useful herein include those in which the amino acid sequences comprise a sequence sharing at least 70% amino acid sequence homology or "similarity", and preferably 80% homology or similarity, with a reference morphogenic protein selected from the foregoing naturally occurring proteins. Preferably, the reference protein is human OP-1, and the reference sequence thereof is the C-terminal seven cysteine domain present in morphogenically active forms of human OP-1, residues 330-431 of SEQ ID NO: 2. It will be appreciated that other known morphogenic proteins also can be used as the reference sequence. In one embodiment, a candidate amino acid sequence thought to be functionally equivalent to a reference amino acid sequence can be aligned therewith using the method of Needleman, *et al*. (1970) J. Mol. Biol. 48:443 -453, implemented conveniently by computer programs such as the Align program (DNAstar, Inc.). Internal gaps and amino acid insertions in the candidate sequence are ignored for purposes of calculating the defined relationship, conventionally expressed as a level of amino acid sequence homology or identity, between the candidate and reference sequences.

"Amino acid sequence homology" is understood herein to include both amino acid sequence identity and similarity. Homologous sequences share identical and/or similar amino acid residues, where similar residues are conservative substitutions for, or "allowed point mutations" of, corresponding amino acid residues in an aligned reference sequence. Thus, a candidate polypeptide sequence that shares 70% amino acid homology with a reference sequence is one in which any 70% of the aligned residues are either identical to, or are conservative substitutions of, the corresponding residues in a reference sequence. Certain particularly preferred morphogenic polypeptide chains share at least 60% amino acid sequence identity with the C terminal seven cysteine domain of the preferred reference sequence of human OP-1, still more preferably at least 65% amino acid sequence identity therewith.

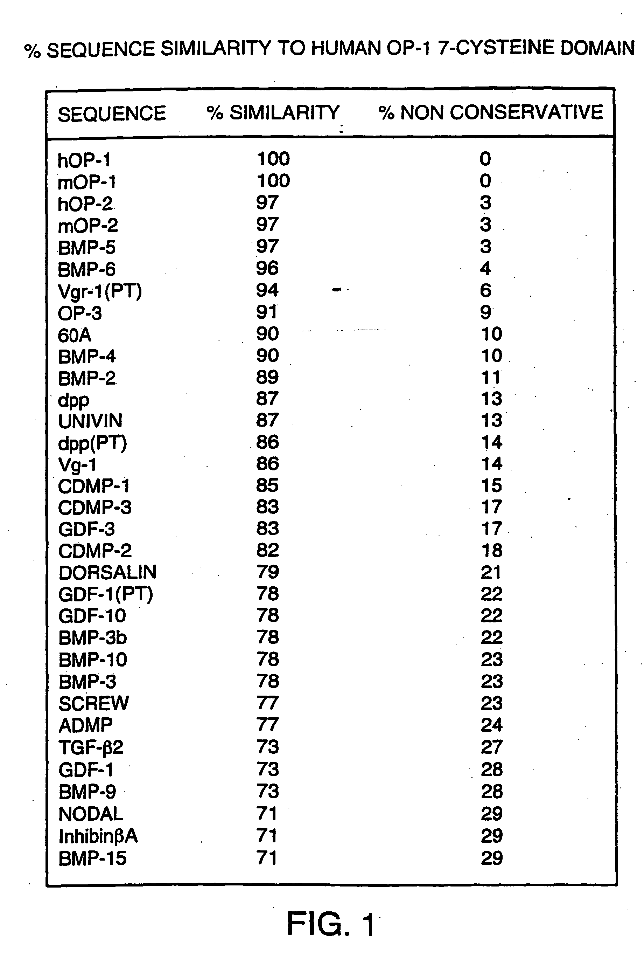

FIG. 1 recites the percent amino acid sequence homology (similarity) and percent identity within the C-terminal seven cysteine domain of various representative members of the TGF-β family, using OP-1 as the reference sequence. The percent homologies recited in the figure are calculated with the sequences aligned essentially following the method of Needleman, *et al*. (1970) *J. Mol Biol, 48:* 443-453, calculated using the Align Program (DNAstar, Inc.). Insertions and deletions from the reference morphogen sequence, here the C-terminal, biologically active seven-cysteine domain or skeleton of hOP-1, are ignored for purposes of calculation.

As is apparent to one of ordinary skill in the art reviewing the sequences for the proteins listed in FIG 1, significant amino acid changes can be made from the reference sequence while retaining morphogenic activity. For example, while the GDF-1 protein sequence shares only about 50% amino acid identity with the hOP-1 sequence described herein, the GDF-1 sequence shares greater than 70% amino acid sequence homology with the hOP-1 sequence, where "homology" is as defined above. Moreover, GDF-1 contains a four amino acid insert (Gly-Gly-Pro-Pro) between the two residues corresponding to residue 372 and 373 of OP-1 (SEQ ID NO: 2). Similarly, BMP-3 has a "extra" residue, a valine, inserted between the two residues corresponding to residues 385 and 386 of hOP-1 (SEQ ID NO: 2). Also, BMP-2 and BMP-4 both are "missing" the amino acid residue corresponding to residue 389 of OP-1 (SEQ ID NO: 2). None of these "deviations" from the reference sequence appear to interfere with biological activity.

As will be understood by those skilled in the art, homologous or functionally equivalent sequences include functionally equivalent arrangements of the cysteine residues within the conserved cysteine skeleton, including amino acid insertions or deletions which alter the linear arrangement of these cysteines, but do not materially impair their relationship in the folded structure of the dimeric protein, including their ability to form such intra- or inter-chain disulfide bonds as may be necessary for biological activity. For example, naturally occurring morphogens have been described in which at least one internal deletion (of one residue; BMP2) or insertion (of four residues; GDF-1) is present but does not abrogate biological activity. Functionally equivalent sequences further include those wherein one or more amino acid residues differ from the corresponding residue of a reference sequence, *e*.*g*., the C-terminal seven cysteine domain (also referred to herein as the conserved seven cysteine skeleton) of human OP-1, provided that this difference does not destroy tissue morphogenic activity.

As used herein, "conservative substitutions" are residues that are physically or functionally similar to the corresponding reference residues, e.g., that have similar size, shape, electric charge, chemical properties including the ability to form covalent or hydrogen bonds, or the like. Particularly preferred conservative substitutions are those fulfilling the criteria defined for an accepted point mutation in Dayhoff *et al*. (1978), 5 Atlas of Protein Sequence and Structure, Suppl. 3, ch. 22 (pp. 354-352), Natl. Biomed. Res. Found., Washington, D.C. 20007. Examples of conservative substitutions include the substitution of one amino acid for another with similar characteristics, *e*.*g*., substitutions within the following groups are well-known: (a) valine, glycine; (b) glycine, alanine; (c) valine, isoleucine, leucine; (d) aspartic acid, glutamic acid; (e) asparagine, glutamine; (f) serine, threonine; (g) lysine, arginine, methionine; and (h) phenylalanine, tyrosine. The term "conservative variant" or "conservative variation" also includes the use of a substituted amino acid in place of an unsubstituted parent amino acid in a given polypeptide chain, provided that antibodies having binding specificity for the resulting substituted polypeptide chain also have binding specificity (*i*.*e*., "crossreact" or "immunoreact" with) the unsubstituted or parent polypeptide chain.

In other preferred embodiments, the family of morphogenic proteins useful in the present invention, and members thereof, are defined by a generic amino acid sequence. For example, Generic Sequence 7 (SEQ ID NO: 4) and Generic Sequence 8 (SEQ ID NO: 5) disclosed below, accommodate the homologies shared among preferred protein family members identified to date, including at least OP-1, OP-2, OP-3, CBMP-2A, CBMP-2B, BMP-3, 60A, DPP, Vgl, BMP-5, BMP-6, Vgr-1, and GDF-1. The amino acid sequences for these proteins are described herein and/or in the art, as summarized above. The generic sequences include both the amino acid identity shared by these sequences in the C-terminal domain, defined by the six and seven cysteine skeletons (Generic Sequences 7 and 8, respectively), as well as alternative residues for the variable positions within the sequence. The generic sequences provide an appropriate cysteine skeleton where inter- or intramolecular disulfide bonds can form, and contain certain specified amino acids that may influence the tertiary structure of the folded proteins. In addition, the generic sequences allow for an additional cysteine at position 36 (Generic Sequence 7) or position 41 (Generic Sequence 8), thereby encompassing the biologically active sequences of OP-2 and OP-3. 
Generic Sequence 7
Leu Xaa Xaa Xaa Phe Xaa Xaa Xaa Gly Trp Xaa Xaa Xaa Xaa Xaa Xaa
1               5                   10                  15
Pro Xaa Xaa Xaa Xaa Ala Xaa Tyr Cys Xaa Gly Xaa Cys Xaa Xaa Pro Xaa Xaa
            20                  25                  30
Xaa Xaa Xaa Xaa Xaa Xaa Asn His Ala Xaa Xaa Xaa Xaa Xaa Xaa Xaa Xaa Xaa
35                  40                  45                  50
Xaa Xaa Xaa Xaa Xaa Xaa Xaa Xaa Cys Cys Xaa Pro Xaa Xaa Xaa Xaa Xaa Xaa
        55                  60                  65                  70
Xaa Xaa Leu Xaa Xaa Xaa Xaa Xaa Xaa Xaa Val Xaa Leu Xaa Xaa Xaa Xaa Xaa
 
                75                  80                  85
Met Xaa Val Xaa Xaa Cys Xaa Cys Xaa
    90                  95
 wherein each Xaa independently is selected from a group of one or more specified amino acids defined as follows: "Res." means "residue" and Xaa at res.2 = (Tyr or Lys); Xaa at res.3 = Val or Ile); Xaa at res.4 = (Ser, Asp or Glu); Xaa at res.6 = (Arg, Gln, Ser, Lys or Ala); Xaa at res.7 = (Asp or Glu); Xaa at res.8 = (Leu, Val or Ile); Xaa at res.11 = (Gln, Leu, Asp, His, Asn or Ser); Xaa at res.12 = (Asp, Arg, Asn or Glu); Xaa at res. 13 = (Trp or Ser); Xaa at res. 14 = (Ile or Val); Xaa at res. 15 = (Ile or Val); Xaa at res. 16 (Ala or Ser); Xaa at res. 18 = (Glu, Gln, Leu, Lys, Pro or Arg); Xaa at res.19 = (Gly or Ser); Xaa at res.20 = (Tyr or Phe); Xaa at res.21 = (Ala, Ser, Asp, Met, His, Gln, Leu or Gly); Xaa at res.23 = (Tyr, Asn or Phe); Xaa at res.26 = (Glu, His, Tyr, Asp, Gln, Ala or Ser); Xaa at res.28 = (Glu, Lys, Asp, Gln or Ala); Xaa at res.30 = (Ala, Ser, Pro, Gln, Ile or Asn); Xaa at res.31 = (Phe, Leu or Tyr); Xaa at res.33 = (Leu, Val or Met); Xaa at res.34 = (Asn, Asp, Ala, Thr or Pro); Xaa at res.35 = (Ser, Asp, Glu, Leu, Ala or Lys); Xaa at res.36 = (Tyr, Cys, His, Ser or Ile); Xaa at res.37 = (Met, Phe, Gly or Leu); Xaa at res.38 = (Asn, Ser or Lys); Xaa at res.39 = (Ala, Ser, Gly or Pro); Xaa at res.40 = (Thr, Leu or Ser); Xaa at res.44 = (Ile, Val or Thr); Xaa at res.45 = (Val, Leu, Met or Ile); Xaa at res.46 = (Gln or Arg); Xaa at res.47 = (Thr, Ala or Ser); Xaa at res.48 = (Leu or Ile); Xaa at res.49 = (Val or Met); Xaa at res.50 = (His, Asn or Arg); Xaa at res.51 = (Phe, Leu, Asn, Ser, Ala or Val); Xaa at res.52 = (Ile, Met, Asn, Ala, Val, Gly or Leu); Xaa at res.53 = (Asn, Lys, Ala, Glu, Gly or Phe); Xaa at res.54 = (Pro, Ser or Val); Xaa at res.55 = (Glu, Asp, Asn, Gly, Val, Pro or Lys); Xaa at res.56 = (Thr, Ala, Val, Lys, Asp, Tyr, Ser, Gly, Ile or His); Xaa at res.57 = (Val, Ala or De); Xaa at res.58 = (Pro or Asp); Xaa at res.59 (Lys, Leu or Glu); Xaa at res.60 = (Pro, Val or Ala); Xaa at res.63 = (Ala or Val); Xaa at res.65 (Thr, Ala or Glu); Xaa at res.66 (Gln, Lys, Arg or Glu); Xaa at res.67 = (Leu, Met or Val); Xaa at res.68 = (Asn, Ser, Asp or Gly); Xaa at res.69 = (Ala, Pro or Ser); Xaa at res.70 = (Ile, Thr, Val or Leu); Xaa at res.71 = (Ser, Ala or Pro); Xaa at res.72 = (Val, Leu, Met or Ile); Xaa at res.74 = (Tyr or Phe); Xaa at res.75 = (Phe, Tyr, Leu or His); Xaa at res.76 = (Asp, Asn or Leu); Xaa at res.77 = (Asp, Glu, Asn, Arg or Ser); Xaa at res.78 = (Ser, Gln, Asn, Tyr or Asp); Xaa at res.79 = (Ser, Asn, Asp, Glu or Lys); Xaa at res.80 = (Asn, Thr or Lys); Xaa at res-82 = (Ile, Val or Asn); Xaa at res.84 = (Lys or Arg); Xaa at res.85 = (Lys, Asn, Gln, His, Arg or Val); Xaa at res.86 = (Tyr, Glu or His); Xaa at res.87 = (Arg, Gln, Glu or Pro); Xaa at res.88 = (Asn, Glu, Trp or Asp); Xaa at res.90 = (Val, Thr, Ala or Ile); Xaa at res-92 = (Arg, Lys, Val, Asp, Gln or Glu); Xaa at res.93 = (Ala, Gly, Glu or Ser); Xaa at res.95 = (Gly or Ala) and Xaa at res.97 = (His or Arg).

Generic Sequence 8 (SEQ ID NO: 5) includes all of Generic Sequence 7 and in addition includes the following five amino acid sequences (SEQ ID NO: 8) at its N-terminus: 
Cys Xaa Xaa Xaa Xaa
1               5
 Accordingly, beginning with residue 7, each "Xaa" in Generic Sequence 8 is a specified amino acid defined as for Generic Sequence 7, with the distinction that each residue number described for Generic Sequence 7 is shifted by five in Generic Sequence 8. Thus, "Xaa at res.2 =(Tyr or Lys)" in Generic Sequence 7 refers to Xaa at res. 7 in Generic Sequence 8. In Generic Sequence 8, Xaa at res.2 = (Lys, Arg, Ala or Gln); Xaa at res.3 = (Lys, Arg or Met); Xaa at res.4 = (His, Arg or Gln); and Xaa at res.5 = (Glu, Ser, His, Gly, Arg, Pro, Thr, or Tyr).

In another embodiment, useful morphogenic proteins include those defined by Generic Sequences 9 and 10, defined as follows.

Specifically, Generic Sequences 9 and 10 are composite amino acid sequences of the following proteins: human OP-1, human OP-2, human OP-3, human BMP-2, human BMP-3, human BMP-4, human BMP-5, human BMP-6, human BMP-8, human BMP-9, human BMP 10, human BMP11, Drosophila 60A, Xenopus Vg-1, sea urchin UNIV11V, human CDMP-1 (mouse GDF-5), human CDMP-2 (mouse GDF-6, human BMP-13), human CDMP-3 (mouse GDF-7, human BMP-12), mouse GDF-3, human GDF- 1, mouse GDF-1, chicken DORSALIN, dpp, Drosophila SCREW, mouse NODAL, mouse GDF-8, human GDF-8, mouse GDF-9, mouse GDF-10, human GDF-11, mouse GDF-11, human BMP-15, and rat BMP3b. Like Generic Sequence 7, Generic Sequence 9 accommodates the C-terminal six cysteine skeleton and, like Generic Sequence 8, Generic Sequence 10 accommodates the seven cysteine skeleton. 
Generic Sequence 9 (SEQ ID NO: 6)
Xaa Xaa Xaa Xaa Xaa Xaa Xaa Xaa Xaa Xaa Xaa Xaa Xaa Xaa Xaa Xaa
1               5                   10                  15
Pro Xaa Xaa Xaa Xaa Xaa Xaa Xaa Cys Xaa Gly Xaa Cys Xaa Xaa Xaa Xaa
            20                  25                  30
Xaa Xaa Xaa Xaa Xaa Xaa Xaa Xaa Xaa Xaa Xaa Xaa Xaa Xaa Xaa Xaa Xaa
    35                  40                  45                  50
Xaa Xaa Xaa Xaa Xaa Xaa Xaa Xaa Xaa Xaa Xaa Cys Xaa Pro Xaa Xaa Xaa
                55                  60                  65
Xaa Xaa Xaa Xaa Xaa Leu Xaa Xaa Xaa Xaa Xaa Xaa Xaa Xaa Xaa Xaa Xaa
        70                  75                  80
Xaa Xaa Xaa Xaa Xaa Xaa Xaa Xaa Xaa Cys Xaa Cys Xaa
85                  90                  95
 wherein each Xaa is independently selected from a group of one or more specified amino acids defined as follows: "Res." means "residue" and Xaa at res. 1 = (Phe, Leu or Glu); Xaa at res. 2 = (Tyr, Phe, His, Arg, Thr, Lys, Gln, Val or Glu); Xaa at res. 3 = (Val, Ile, Leu or Asp); Xaa at res. 4 = (Ser, Asp, Glu, Asn or Phe); Xaa at res. 5 = (Phe or Glu); Xaa at res. 6 = (Arg, Gln, Lys, Ser, Glu, Ala or Asn); Xaa at res. 7 = (Asp, Glu, Leu, Ala or Gln); Xaa at res. 8 = (Leu, Val, Met, Ile or Phe); Xaa at res. 9 = (Gly, His or Lys); Xaa at res. 10 = (Trp or Met); Xaa at res. 11 = (Gln, Leu, His, Glu, Asn, Asp, Ser or Gly); Xaa at res. 12 = (Asp, Asn, Ser, Lys, Arg, Glu or His); Xaa at res. 13 = (Trp or Ser); Xaa at res. 14 = (Ile or Val); Xaa at res. 15 = (Ile or Val); Xaa at res. 16 = (Ala, Ser, Tyr or Trp); Xaa at res. 18 = (Glu, Lys, Gln, Met, Pro, Leu, Arg, His or Lys); Xaa at res. 19 = (Gly, Glu, Asp, Lys, Ser, Gln, Arg or Phe); Xaa at res. 20 = (Tyr or Phe); Xaa at res. 21 = (Ala, Ser, Gly, Met, Gln, His, Glu, Asp, Leu, Asn, Lys or Thr); Xaa at res. 22 = (Ala or Pro); Xaa at res. 23 = (Tyr, Phe, Asn, Ala or Arg); Xaa at res. 24 = (Tyr, His, Glu, Phe or Arg); Xaa at res. 26 = (Glu, Asp, Ala, Ser, Tyr, His, Lys, Arg, Gln or Gly); Xaa at res. 28 = (Glu, Asp, Leu, Val, Lys, Gly, Thr, Ala or Gln); Xaa at res. 30 (Ala, Ser, Ile, Asn, Pro, Glu, Asp, Phe, Gln or Leu); Xaa at res. 31 = (Phe, Tyr, Leu, Asn, Gly or Arg); Xaa at res. 32 = (Pro, Ser, Ala or Val); Xaa at res. 33 = (Leu, Met, Glu, Phe or Val); Xaa at res. 34 = (Asn, Asp, Thr, Gly, Ala, Arg, Leu or Pro); Xaa at res. 35 = (Ser, Ala, Glu, Asp, Thr, Leu, Lys, Gln or His); Xaa at res. 36 = (Tyr, His, Cys, Ile, Arg, Asp, Asn, Lys, Ser, Glu or Gly); Xaa at res. 37 = (Met, Leu, Phe, Val, Gly or Tyr); Xaa at res. 38 = (Asn, Glu, Thr, Pro, Lys, His, Gly, Met, Val or Arg); Xaa at res. 39 = (Ala, Ser, Gly, Pro or Phe); Xaa at res. 40 (Thr, Ser, Leu, Pro, His or Met); Xaa at res. 41 = (Asn, Lys, Val, Thr or Gln); Xaa at res. 42 (His, Tyr or Lys); Xaa at res. 43 = (Ala, Thr, Leu or Tyr); Xaa at res. 44 = (Ile, Thr, Val, Phe, Tyr, Met or Pro); Xaa at res. 45 = (Val, Leu, Met, Ile or His); Xaa at res. 46 = (Gln, Arg or Thr); Xaa at res. 47 = (Thr, Ser, Ala, Asn or His); Xaa at res. 48 = (Leu, Asn or Ile); Xaa at res. 49 (Val, Met, Leu, Pro or Ile); Xaa at res. 50 = (His, Asn, Arg, Lys, Tyr or Gln); Xaa at res: 51 (Phe, Leu, Ser, Asn, Met, Ala, Arg, Glu, Gly or Gln); Xaa at res. 52 = (Ile, Met, Leu, Val, Lys, Gln, Ala or Tyr); Xaa at res. 53 = (Asn, Phe, Lys, Glu, Asp, Ala, Gln, Gly, Leu or Val); Xaa at res. 54 = (Pro, Asn, Ser, Val or Asp); Xaa at res. 55 = (Glu, Asp, Asn, Lys, Arg, Ser, Gly, Thr, Gln, Pro or His); Xaa at res. 56 = (Thr, His, Tyr, Ala, Ile, Lys, Asp, Ser, Gly or Arg); Xaa at res. 57 = (Val, Ile, Thr, Ala, Leu or Ser); Xaa at res. 58 = (Pro, Gly, Ser, Asp or Ala); Xaa at res. 59 = (Lys, Leu, Pro, Ala, Ser, Glu, Arg or Gly); Xaa at res. 60 = (Pro, Ala, Val, Thr or Ser); Xaa at res. 61 = (Cys, Val or Ser); Xaa at res. 63 = (Ala, Val or Thr); Xaa at res. 65 = (Thr, Ala, Glu, Val, Gly, Asp or Tyr); Xaa at res. 66 = (Gln, Lys, Glu, Arg or Val); Xaa at res. 67 = (Leu, Met, Thr or Tyr); Xaa at res. 68 = (Asn, Ser, Gly, Thr, Asp, Glu, Lys or Val); Xaa at res. 69 = (Ala, Pro, Gly or Ser); Xaa at res. 70 = (Ile, Thr, Leu or Val); Xaa at res. 71 = (Ser, Pro, Ala, Thr, Asn or Gly); Xaa at res. 2 = (Val, Ile, Leu or Met); Xaa at res. 74 = (Tyr; Phe, Arg, Thr, Tyr or Met); Xaa at res. 75 = (Phe, Tyr, His, Leu, Ile, Lys, Gln or Val); Xaa at res. 76 = (Asp, Leu, Asn or Glu); Xaa at res. 77 = (Asp, Ser, Arg, Asn, Glu, Ala, Lys, Gly or Pro); Xaa at res. 78 = (Ser, Asn, Asp, Tyr, Ala, Gly, Gln, Met, Glu, Asn or Lys); Xaa at res. 79 = (Ser, Asn, Glu, Asp, Val, Lys, Gly, Gln or Arg); Xaa at res. 80 = (Asn, Lys, Thr, Pro, Val, Ile, Arg, Ser or Gln); Xaa at res. 81 = (Val, Ile, Thr or Ala); Xaa at res. 82 = (Ile, Asn, Val, Leu, Tyr, Asp or Ala); Xaa at res. 83 = (Leu, Tyr, Lys or Ile); Xaa at res. 84 = (Lys, Arg, Asn, Tyr, Phe, Thr, Glu or Gly); Xaa at res. 85 = (Lys, Arg, His, Gln, Asn, Glu or Val); Xaa at res. 86 = (Tyr, His, Glu or Ile); Xaa at res. 87 = (Arg, Glu, Gln, Pro or Lys); Xaa at res. 88 = (Asn, Asp, Ala, Glu, Gly or Lys); Xaa at res. 89 = (Met or Ala); Xaa at res. 90 = (Val, Ile, Ala, Thr, Ser or Lys); Xaa at res 91 (Val or Ala); Xaa at res. 92 = (Arg, Lys, Gln, Asp, Glu, Val, Ala, Ser or Thr); Xaa at res. 93 (Ala, Ser, Glu, Gly, Arg or Thr); Xaa at res. 95 = (Gly, Ala or Thr); Xaa at res. 97 = (His, Arg, Gly, Leu or Ser). Further, after res. 53 in rBMP3b and mGDF-10 there is an Ile; after res. 54 in GDF-1 there is a T; after res. 54 in BMP there is a V; after res. 78 in BMP-8 and Dorsalin there is a G; after res. 37 in hGDF-1 there is Pro, Gly, Gly, Pro.

Generic Sequence 10 (SEQ ID NO: 7) includes all of Generic Sequence 9 (SEQ ID NO: 6) and in addition includes the following sequence (SEQ ID NO: 9) at its N-terminus: 
SEQ ID NO: 10
Cys Xaa Xaa Xaa Xaa
1               5
 Accordingly, beginning with residue 6, each "Xaa" in Generic Sequence 10 is a specified amino acid defined as for Generic Sequence 9, with the distinction that each residue number described for Generic Sequence 9 is shifted by five in Generic Sequence 10. Thus, "Xaa at res. 1 = (Tyr, Phe, His, Arg, Thr, Lys, Gln, Val or Glu)" in Generic Sequence 9 refers to Xaa at res. 6 in Generic Sequence 10. In Generic Sequence 10, Xaa at res. 2 = (Lys, Arg, Gln, Ser, His, Glu, Ala, or Cys); Xaa at res. 3 = (Lys, Arg, Met, Lys, Thr, Leu, Tyr, or Ala); Xaa at res. 4 = (His, Gln, Arg, Lys, Thr, Leu, Val, Pro, or Tyr); and Xaa at res. 5 = (Gln, Thr, His, Arg, Pro, Ser, Ala, Gln, Asn, Tyr, Lys, Asp, or Leu).

As noted above, certain currently preferred bone morphogenic polypeptide sequences useful in this invention have greater than 60% identity, preferably greater than 65% identity, with the amino acid sequence defining the preferred reference sequence of hOP-1. These particularly preferred sequences include allelic and phylogenetic counterpart variants of the OP-1 and OP-2 proteins, including the Drosophila 60A protein. Accordingly, in certain particularly preferred embodiments, useful proteins include active proteins comprising pairs of polypeptide chains within the generic amino acid sequence herein referred to as "OPX" (SEQ ID NO: 3), which defines the seven cysteine skeleton and accommodates the homologies between several identified variants of OP-1 and OP-2. As described herein, each Xaa at a given position independently is selected from the residues occurring at the corresponding position in the C-terminal sequence of mouse or human OP-1 or OP-2. 
Cys Xaa Xaa His Glu Leu Tyr Val Ser Phe Xaa Asp Leu Gly Trp Xaa Asp Trp
1               5                   10                  15
Xaa Ile Ala Pro Xaa Gly Tyr Xaa Ala Tyr Tyr Cys Glu Gly Glu Cys Xaa Phe Pro
    20                  25                  30                  35
Leu Xaa Ser Xaa Met Asn Ala Thr Asn His Ala Ile Xaa Gln Xaa Leu Val His Xaa
        40                  45                  50                  55
Xaa Xaa Pro Xaa Xaa Val Pro Lys Xaa Cys Cys Ala Pro Thr Xaa Leu Xaa Ala
            60                  65                  70
Xaa Ser Val Leu Tyr Xaa Asp Xaa Ser Xaa Asn Val Ile Leu Xaa Lys Xaa Arg
75 809 85 90
Asn Met Val Val Xaa Ala Cys Gly Cys His
        95                  100
 wherein Xaa at res. 2 = (Lys or Arg); Xaa at res. 3 = (Lys or Arg); Xaa at res. 11 = (Arg or Gln); Xaa at res. 16 = (Gln or Leu); Xaa at res. 19 = (Ile or Val); Xaa at res. 23 = (Glu or Gln); Xaa at res. 26 = (Ala or Ser); Xaa at res. 35 = (Ala or Ser); Xaa at res. 39 = (Asn or Asp); Xaa at res: 41 = (Tyr or Cys); Xaa at res. 50 = (Val or Leu); Xaa at res. 52 = (Ser or Thr); Xaa at res. 56 = (Phe or Leu); Xaa at res. 57 = (Ile or Met); Xaa at res. 58 = (Asn or Lys); Xaa at res. 60 = (Glu, Asp or Asn); Xaa at res. 61 =(Thr, Ala or Val); Xaa at res. 65 = (Pro or Ala); Xaa at res. 71 = (Gln or Lys); Xaa at res. 73 =(Asn or Ser); Xaa at res. 75 = (Ile or Thr); Xaa at res. 80 = (Phe or Tyr); Xaa at res. 82 = (Asp or Ser); Xaa at res. 84 = (Ser or Asn); Xaa at res. 89 = (Lys or Arg); Xaa at res. 91 =(Tyr or His); and Xaa at res. 97 = (Arg or Lys).

In still another preferred embodiment, useful morphogenically active proteins have polypeptide chains with amino acid sequences comprising a sequence encoded by a nucleic acid that hybridizes, under low, medium or high stringency hybridization conditions, to DNA or RNA encoding reference morphogenic sequences, *e*.*g*., C-terminal sequences defining the conserved seven cysteine domains of OP-1, OP-2, BMP2, BMP4, BMP5, BMP6, 60A, GDF5, GDF6, GDF7 and the like. As used herein, high stringent hybridization conditions are defined as hybridization according to known techniques in 40% formamide, 5 X SSPE, 5 X Denhardt's Solution, and 0.1 % SDS at 37°C overnight, and washing in 0.1 X SSPE, 0.1 % SDS at 50°C. Standard stringency conditions are well characterized in commercially available, standard molecular cloning texts. See, for example, *Molecular Cloning A Laboratory Manual*, 2nd Ed., ed. by Sambrook, Fritsch and Maniatis (Cold Spring Harbor Laboratory Press: 1989); *DNA Cloning*, Volumes I and II (D.N. Glover ed., 1985); *Oligonucleotide Synthesis* (M.J. Gait ed., 1984): *Nucleic Acid Hybridization* (B. D. Hames & S.J. Higgins eds. 1984); and B. Perbal, *A Practical Guide To Molecular Cloning* (1984).

The morphogenic proteins contemplated herein can be expressed from intact or truncated genomic or cDNA or from synthetic DNAs in prokaryotic or eukaryotic host cells. The dimeric proteins can be isolated from the culture media and/or refolded and dimerized *in vitro* to form biologically active compositions. Heterodimers can be formed *in vitro* by combining separate, distinct polypeptide chains. Alternatively, heterodimers can be formed in a single cell by co-expressing nucleic acids encoding separate, distinct polypeptide chains. See, for example, W093/09229, or U.S. Patent No. 5,411,941, for several exemplary recombinant heterodimer protein production protocols. Currently preferred host cells include, without limitation, prokaryotes including *E. coli*, or eukaryotes including yeast or Saccharomyces, or mammalian cells, such as CHO, COS or BSC cells. One of ordinary skill in the art will appreciate that other host cells can be used to advantage. Detailed descriptions of the proteins useful in the practice of this invention, including how to make, use and test them for morphogenic activity, are disclosed in numerous publications, including US Patent Nos. 5,266,683, 5,011,691,and/or U.S. Patent No. 5,585,237, the disclosures of which are incorporated by reference herein, as well as in any of the publications recited herein, including any of the U.S. patents, the disclosures of which are incorporated herein by reference.

The dimeric protein species described herein above are referred to herein as "mature" morphogenic proteins. The mature dimeric protein can be administered to a tissue or a physiological fluid alone, or complexed with at least one strand of its pro domain to create a "soluble complex". Soluble complex forms of these proteins can be created by complexing the dimeric species with part or all of at least one, and preferably two morphogenic protein pro domain peptides. Alternatively, a soluble complex form of a morphogenic protein can be isolated from the cell culture media using the protocol described in W094/03600, published 18 Feb 1994, for example. (See below).

Other soluble complex forms of morphogens include dimers of the uncleaved pro forms of these proteins, as well as "hemi-dimers" wherein one subunit of the dimer is an uncleaved pro form of the protein, and the other subunit comprises the mature form of the protein, including truncated forms thereof, preferably noncovalently associated with a cleaved pro domain peptide.

As described in published application W094/03600, the teachings of which are incorporated herein by reference, useful pro domains include the full length pro regions, as well as various truncated forms thereof, particularly truncated forms cleaved at proteolytic Arg-Xaa-Xaa-Arg cleavage sites within the pro domain polypeptide. For example, in OP1, possible pro sequences include sequences defined by residues 30-292 (full length form); 48-292; and 158-292, all of SEQ ID NO: 2. Soluble OP1 complex stability is best enhanced when the pro region comprises the full length form rather than a truncated form, such as the residues 48-292 truncated form, in that residues 30-47 show sequence homology to the N-terminal portions of other morphogens, and currently are believed to have particular utility in enhancing complex stability for all morphogens. Accordingly, currently preferred pro domains include peptides comprising at least the N-terminal fragment, *e*.*g*., amino acid residues 30-47 of a naturally occurring morphogen pro domain, or a biosynthetic variant thereof that retains the solubility and/or stability enhancing properties of the naturally-occurring peptide.

As will be appreciated by those having ordinary skill in the art, useful sequences encoding the pro region can be obtained from genetic sequences encoding known morphogens. Alternatively, chimeric pro regions can be constructed from the sequences of one or more known morphogens. Still another option is to create a synthetic sequence variant of one or more known pro region sequences.

Soluble morphogen complexes can be isolated from conditioned media using a simple, three step chromatographic protocol performed in the absence of denaturants. The protocol involves running the media (or body fluid) over an affinity column, followed by ion exchange and gel nitration chromatographies. The affinity column described below is a Zn-IMAC column. An alternative protocol also envisioned to have utility includes an immunoaffinity column, created using standard procedures and, for example, using antibody specific for a given morphogen pro domain (complexed, for example, to a protein A-conjugated Sepharose column). Protocols for developing immunoaffinity columns are well described in the art (see, for example, Guide to Protein Purification, M. Deutscher, ed., Academic Press, San Diego, 1990, particularly sections VII and XI thereof).

Morphogens can be expressed in any suitable host cell competent to express recombinant protein. For example, mammalian (CHO, Chinese hamster ovary) cells as described in the art (see, for example, international application US90/05903 (WO91/05802) can be used. The CHO cell conditioned media containing 0.5% FBS is initially purified using Immobilized Metal-Ion Affinity Chromatography (IMAC). The soluble morphogen complex from conditioned media binds very selectively to the Zn-IMAC resin and a high concentration of imidazole (50 mM imidazole, pH 8.0) is required for the effective elution of the bound complex. The Zn-IMAC step separates the soluble morphogen from the bulk of the contaminating serum proteins that elute in the flowthrough and 35 mM imidazole wash fractions. The Zn-IMAC purified soluble morphogen is next applied to an S-Sepharose cation-exchange column equilibrated in 20 mM NaPO₄ (pH 7.0) with 50 mM NaCl. This S-Sepharose step serves to further purify and concentrate the soluble complex in preparation for the following gel filtration step. The protein was applied to a Sephacryl S-200HR column equilibrated in TBS. Using substantially the same protocol, soluble morphogens also can be isolated from one or more body fluids, including serum, cerebrospinal fluid or peritoneal fluid.

The soluble complex isolated from cell culture media elutes with an apparent molecular weight of 110 kDa as compared with protein molecular weight standards. The identity of the proteins can be confirmed by N-terminal sequencing. Purity of the final complex can be verified by running the appropriate fraction in a reduced 15% polyacrylaniide gel.

As an alternative to purifying soluble complexes from culture media or a body fluid, soluble complexes can be formulated from purified pro domains and mature dimeric species. Successful complex formation apparently requires association of the components under denaturing conditions sufficient to relax the folded structure of these molecules, without affecting disulfide bonds. Preferably, the denaturing conditions mimic the environment of an intracellular vesicle sufficiently such that the cleaved pro domain has an opportunity to associate with the mature dimeric species under relaxed folding conditions. The concentration of denaturant in the solution then is decreased in a controlled, preferably step-wise manner, so as to allow proper refolding of the dimer and pro regions while maintaining the association of the pro domain with the dimer. Useful denaturants include 4-6M urea or guanidine hydrochloride (GuHCI), in buffered solutions of pH 4-10, preferably pH 6-8. The soluble complex then is formed by controlled dialysis or dilution into a solution having a final denaturant concentration of less than 0.1-2M urea or GuHCI, preferably 1-2 M urea of GuHCI, which then preferably can be diluted into a physiological buffer. Protein purification/renaturing procedures and considerations are well described in the art, and details for developing a suitable renaturing protocol readily can be determined by one having ordinary skill in the art. One useful text on the subject is Guide to Protein Purification, M. Deutscher, ed., Academic Press, San Diego, 1990, particularly section V. Complex formation also may be aided by addition of one or more chaperone proteins. Stability of the complex also is enhanced by presence of positively charged amino atids, such as arginine.

### B. Properties of Nucleic Acids for Administration

Alternatively, a nucleic acid encoding the morphogen of choice can be provided to the mammal. For example, "naked" nucleic acid, that is, unassociated with a viral particle or other delivery vehicle that can facilitate entry into the cell, including liposomal formulations, charged lipids and/or precipitating agents such as CaPO₄, can be used to advantage. See, for example, US Patent No. 5,580,859, to Felgner et al., the disclosure of which is incorporated herein by reference.

Briefly, a polynucleotide ("nucleic acid") operatively codes for a polypeptide when it has all the genetic information necessary for expression by a target cell, such as promoters and the like. These polynucleotides can be administered to an animal, including a human, by any method that delivers injectable materials to cells of the animal, such as by injection into the interstitial space of tissues such as muscles or skin, introduction into the circulation or into body cavities, or by inhalation or insufflation. A naked polynucleotide is injected or otherwise delivered to the animal with a pharmaceutically acceptable liquid carrier. In preferred applications, the liquid carrier is aqueous or partly aqueous, comprising sterile, pyrogen-free water. The pH of the preparation is suitably adjusted and buffered. The polynucleotide can comprise a complete gene, a fragment of a gene, or several genes, together with recognition and other sequences necessary for expression.

Where the polynucleotide is to be DNA, promoters suitable for use in various vertebrate systems are well known. For example, for use in murine systems, suitable strong promoters include RSV LTR, MPSV LTR, SV40 IEP and metallothionein promoter. In humans, on the other hand, promoters such as CMV IEP may advantageously be used. All forms of DNA, whether replicating or non-replicating, which do not become integrated into the genome, and which are expressible, are within the methods contemplated by the invention.

Non-replicating DNA sequences can be introduced into cells to provide production of the desired polypeptide for periods of about up to six months. Alternatively, an even more prolonged effect can be achieved by introducing the DNA sequence into the cell by means of a vector plasmid having the DNA sequence inserted therein. Preferably, the plasmid further comprises a replicator. Such plasmids are well known to those skilled in the art, for example, plasmid pBR322, with replicator pMB1, or plasmid pMK16, with replicator ColE1 (Ausubel, *C-urrent Protocols in Molecular Biology*, John Wiley and Sons, New York (1988) II: 1.5.2.

In the embodiments of the invention where use of liposomes is desired as, for example, when the polynucleotide is to be associated with a liposome, a material for forming liposomes, preferably cationic or positively charged liposomes are required, and liposomal preparations are made from these materials. With the liposomal material in hand, the polynucleotide may advantageously be used to administer polynucleotides into bodily sites where liposomes may be taken up by phagocytic cells.

Preferably, the liposomal preparation also comprises both DNA and an RNA polymerase, such as the phage polymerases T7, T3 and SP6. The liposome also includes an initial source of the appropriate RNA polymerase, by either including the actual enzyme itself, or alternatively, an mRNA coding for that enzyme. When the liposome is introduced into the organism, it delivers the DNA and the initial source of RNA polymerase to the cell. The RNA polymerase, recognizing the promoters on the introduced DNA, transcribes both genes, resulting in translation products comprising more RNA polymerase and the desired polypeptide. Production of these materials continues until the introduced DNA (which is usually in the form of a plasmid) is degraded. In this manner, production of the desired polypeptide in vivo can be achieved in a few hours and be extended for one month or more.

Alternatively, viral vector-driven constructs also can be used to advantage. For example, adenovirus vectors have the advantage of transient gene expression (lasting days or weeks) and good transfection efficiency into non-replicating cells. Alternatively, a neurotrophic viral vector, such as herpes virus can be used to advantage. Construction of replication-deficient viruses is well-characterized. See, for example, Kanege, et. al. (1994) *Jpn J. Med Sci Biol. 47:* 157-166 or Ooboshi, et al. (1995) *Cir. Res* 77:7-13. Briefly, the DNA constructs typically comprise a full-length copy of the adenovirus genome of ≈ 37.5 kb, from which the early region I (El) genes are replaced by cDNA for the gene of interest, here, a morphogen, *e.g.*, OP-1 (Seq. ID No. 1) preceded by a simian virus 40 (SV 40) nuclear localization signal in Ad2/CMV-βGal (where CMV is cytomegalovirus). Recombinant viruses are grown in a suitable cell, typically human embryonic kidney (293) cells that complement the El early viral promoters. Virus titer (infectious unit) is determined by using anti-adenovirus antibody, and the virus is suspended in PBS with 20% sucrose and is kept at -70°C until use.

### C. Morphogen Analogs

It will be appreciated by the skilled artisan that, in addition, to administering a morphogen protein or the DNA encoding the morphogen, any molecule competent to induce a "morphogen-mediated biological effect" can be used to advantage. As used herein, a "morphogen-mediated biological effect" is any biological effect resulting from exposure to, or contact of, morphogen-responsive cells or tissue with a morphogen, whether *in vitro* or *in vivo*. A morphogen-mediated biological effect of particular interest herein includes stimulating the expression of one or more phenotype-specific genes, including affecting (*e*.*g*., stimulating or inhibiting) expression of a gene product involved in regulating the expression ofa phenotype-specific gene. So, as used herein, the biological effect of interest can be mediated by a variety of mechanisms, including, for example, binding to a morphogen cell surface receptor and binding to, or stimulating the binding of, an intracellular substance to a protein or DNA sequence involved in gene expression. Preferred morphogen-mediated biological effects include maintenance of a differentiated phenotype, or induction of redifferentiation, and/or stimulation of cellular proliferation and cellular differentiation. Most preferably, the phenotype is correlated with cognitive function. Examples of morphogen-mediated biological effects, including cellular and molecular responses to morphogen exposure, are described in co-owned, co-pending patent applications, 08/115,914, 08/155,343, 08/260,675, 08/165,541 and 08/174,605, the disclosures of which are incorporated by reference herein.

Thus, as used herein, a morphogen analog is any substance that mimics morphogen activation of the regulatory pathway of phenotype-specific gene expression, inducing at least one "morphogen-mediated biological effect" in a cell or tissue. Examples include naturally-occuring or synthetic molecules that are peptide-based or non-peptide based analogs, including small molecule mimetics, and antibodies, antibody fragments and single chain antibody binding sites ("BABS") where the epitope binds the receptor binding site. Useful molecules include naturally-occurring or synthetic morphogen homologs or "mimetics", that is, molecules that functionally substitute for a morphogen by binding to the same cell surface receptor binding sites and activating the receptor as a morphogen does. Other useful molecules include those that occur upstream or downstream of morphogen activation in the morphogen-activated regulatory pathway and homologs, analogs or mimetics of these upstream or downstream molecules, and/or molecules that modulate expression of upstream or downstream members of the pathway.

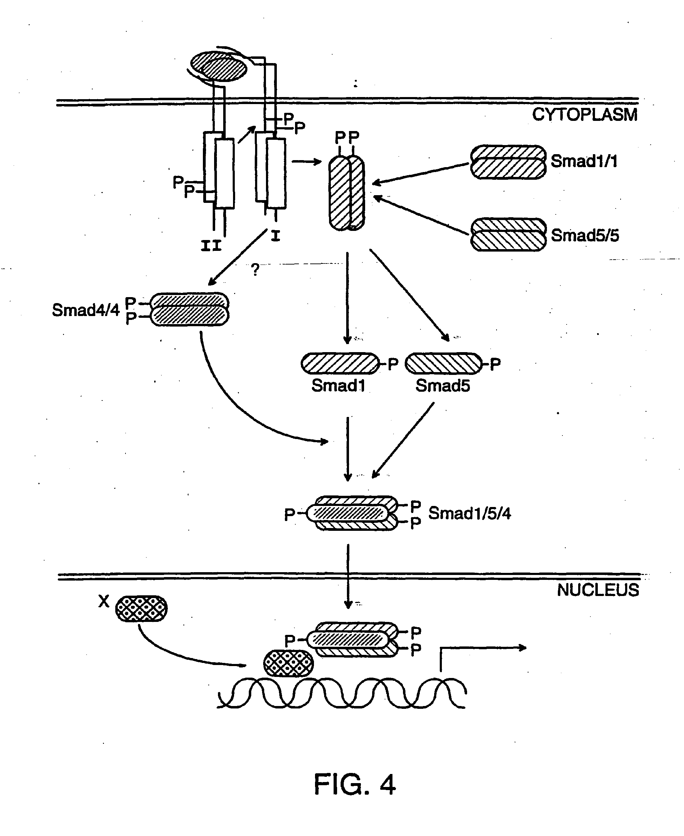

Upstream or downstream molecules include molecules competent to induce endogenous expression of a morphogen, directly or indirectly, and/or molecules that mediate a downstream effect typically mediated by morphogen activation. For example, as exemplified schematically in Fig. 4, the intracellular Smad molecules are activated upon morphogen-receptor binding, and these molecules in turn activate DNA binding molecules to activate gene expression. Specifically, morphogens are ligands for the type I and type II receptors. Morphogen binding to a type I/type II combination activates phosphorylation of the type I receptor by the type-II receptor, and the activated type I receptor specifically phosphorylates Smad 1 homodimers. The type I receptor also specifically phosphorylates Smad5 homodimers. The homodimers then separate to form, in association with a phosphorylated Smad4 molecule, a phosphorylated heteromeric complex comprising at least a Smad1 and a Smad4. A phosphorylated Smad1/Smad5/Smad4 complex alternatively can be formed. The heteromeric complex then translocates into the nucleus, and accumulates therein. In the nucleus, the Smad complex binds operative DNA, either alone or in association with a specific DNA binding protein (the "X-protein") to initiate DNA transcription. The "X-protein acts as a DNA-binding protein, binding the Smad heteromeric complex to the DNA. The pathway leading to endogenous morphogen expression is similar to the one described above, with the Smad heteromeric complex inducing transcription of the morphogen-encoding gene. Other intracellular pathways are induced by morphogens, and can be affected in the manner described herein. Thus, useful molecules include activated Smad hetero- and homodimers and mimetics thereof, functional mimetics of the Smad complex/X-protein combination, molecules that activate one or more Smads, molecules that mediate an effect downstream of Smad, and functional mimetics of these.

A small molecule can be a morphogen analog that mimics activation of the regulatory pathway by a morphogen, such as OP-1. Small molecule morphogen analogs are identified, for example, as reported in co-owned, co-pending patent application, U.S.S.N. 08/507,750, incorporated by reference herein. Any substance having such mimetic properties, regardless of the chemical or biochemical nature thereof, is useful as a morphogen analog as taught herein. The present morphogen analog can be a simple or complex substance produced by a living system or through chemical or biochemical synthetic techniques. It can be a substance that occurs in nature or a novel substance, *e*.*g*., prepared according to principles of rational drug design. It can be a substance that structurally resembles a solvent-exposed morphogen surface epitope implicated in receptor interactions, a substance that otherwise stimulates a transmembrane morphogen receptor, or a cell-membrane permeant substance that interacts with any one or more intracellular aspects of the signal transduction pathway of a morphogen responsive cell. For example, a naturally-sourced OP-1 or morphogen analog can comprise a polypeptide, polynucleotide, carbohydrate, lipid, amino acid, nucteic acid, sugar, fatty acid, steroid, or a derivative of any one of the aforementioned compounds. It can be an intermediate or end product of metabolism of a eukaryotic or prokaryotic cell. Alternatively, the analog can be a biological response modifier or a toxin.

Without being limited, one type of morphogen analog useful in the methods of the present invention can be prepared through application of the principles of biosynthetic antibody binding site (BABS) technology as set forth in U.S. Patent Nos. 5,132,405, 5,091,513 and 5,258,498, the teachings of which are incorporated by reference herein. BABS analog constructs are prepared from antibodies, preferably produced by hybridoma cells, that bind specifically to a morphogen transmembrane receptor. Alternatively, BASS analysis is based upon anti-idiotypic antibodies specifically reactive with the antigen binding site of an antibody that blocks morphogen biological activity. Vukicevic *et al., Biochem. Biophys. Res. Comm. 198:* 693-700 (1994), teaches the preparation of OP-1 specific monoclonal antibodies. Skilled artisans will appreciate that such antibodies can be used as immunogens in the routine preparation of anti-idiotypic antibodies from which BABS analogs of the present invention can be prepared.

A structurally distinct class of morphogen analogs, again set forth herein for illustration and not for limitation, can be prepared through application of the principles of directed molecular evolution as set forth in Tuerk *et al., Science 249:* 505-510 (1990), Famulok *et al., Angew. Chem. Intl. Ed. Engl. 31*:979-988 (1992) and Bock *et al., Nature* 355:564-556 (1992), the teachings of each of which are incorporated by reference herein. The directed molecular evolution process involves isolation of a nucteic acid molecule, for example, an RNA or DNA, or an analog or derivative thereof, particularly one having enhanced stability, that binds with high affinity to a selected ligand such as a protein. Such a nucleic acid molecule is referred to in the art as an "aptamer." The desired aptamer is initially present in a random pool of nucleic acid molecules, and is isolated by performing several rounds of ligand-affinity based chromatography alternating with PCR-based amplification of ligand-binding nucleic acids. Bock et al. (1992), above, have demonstrated the preparations of aptamers, suitable for *in vivo* use in mammals, that specifically inhibit the blood clot promoting factor, thrombin.

Yet another structurally distinct class of morphogen analogs is prepared by selecting appropriate members of a random peptide library (Scott et al. (1990) *Science* 249:386-390), or a combinatorially synthesized random library of organic or inorganic compounds. Needels, *et al*., *Proc. Natl. Acad. Sci. USA, 90:* 10700-10704 (1993); Ohlmeyer *et al, Proc. Natl. Acad. Sci. USA 90*:10922-10926 (1993). Skilled artisans appreciate that the foregoing and other related technologies, taken together with long-established principles of screening biologically-produced substances, offer a wide array of candidate compositions for screening for morphogen analog activity.

One way in which to identify a candidate small molecule is to assay for the ability of the candidate to modulate the effective systemic or local concentration of a morphogen. This can be done, for example, by incubating the candidate in a cell culture that produces the morphogen, and assaying the culture for a parameter indicative of a change in the production level of the morphogen according the methods of U.S.S.N. 08/451,953 and/or U.S. 5,650,276, the teachings of each of which are incorporated by reference herein. Alternatively, candidate compounds are screened for their ability to induce production of a phenotype-specific marker (e.g., a specific nucleic acid, protein, lipid or carbohydrate) in a cell culture in which morphogen activity typically is not present. Examples of compositions which can be screened for their effect on the production of morphogens or phenotype-specific markers include but are not limited to chemicals, biological response modifiers (*e*.*g*., lymphokines, cytokines, hormones, or vitamins), plant extracts, microbial broths and extracts medium conditioned by eukaryotic cells, body fluids, or tissue extracts. Useful candidate compositions then can be tested for *in vivo* efficacy in a suitable animal model. These compositions then can be used *in vivo* to upregulate morphogen-activated regulatory pathways.

A simple method of determining if a small molecule compositions has affected a change in the level of a phenotype-specific protein in cultured cells is provided in co-owned, co-pending patent application, U.S.S.N. 08/451,953, the disclosure of which is incorporated by reference herein. The level of a target phenotype-specific protein in a cell resulting from exposure to a small molecule is measured. Alternatively, a change in the activity or amount of an intracellular pathway component is measured in response to application of a candidate small molecule. Candidates having the desired affect on protein production or pathway regulation are selected for use in methods of the invention. If, for example, a composition upregulates the production of OP-1 by a kidney cell line, it would then be desirable to test systemic administration of this compound in an animal model to determine if it upregulates the production of OP-1 *in vivo*. The level of morphogen in the body can be a result of a wide range of physical conditions, e.g., tissue degeneration such as occurs in diseases including arthritis, emphysema, osteoporosis, kidney diseases, lung diseases, cardiomyopathy, and cirrhosis of the liver. The decrease in level of morphogens in the body can also occur as a result of the normal process of aging. The same strategy is used for compositions affecting intracellular pathway components. A composition selected by these screening methods is then used as a treatment or prophylactic.

### II. FORMULATION AND DELIVERY CONSIDERATIONS

### III. General Considerations

The compositions useful in practice of the invention can be formulated using routine methods. All that is required is determination of the desired final concentration of morphogenic protein per administration. Useful formulation methodologies include solubilization of lyophilized protein. Useful protein solubilization solutions include ethanol, urea, physiological and/or acidic buffers, saline buffers, and acetonitrile/trifluoroacetic acid solutions, and the like. See, for example, U.S. 5,266,683. The desired final concentration of protein will depend on the specific activity of the protein as well as the type, volume, and/or anatomical location of the defect. In one preferred embodiment, useful proteins are those having a half maximal bone forming specific activity of 1.0-2.0 ng protein/25mg matrix, or 0.5-1.0 ng protein/25mg matrix as measured in a standard rat bioassay. Proteins having lower specific activity also can be used to advantage as can morphogen having specific activity in a different tissue morphogens assay. Additionally, the desired final concentration of protein can depend on the age, sex and/or overall health of the recipient.

Useful dosage ranges are contemplated to include 0.001 - 1000 mg/kg body weight, preferably in the range of 0.01 - 100 mg/kg. As described herein below, protein can be administered systemically as a single bolus or as multiple doses administered over time. Useful concentrations for liquid administration include a range from about 0.5-5000 ml. Optimization of dosages requires no more than routine experimentation and is within the skill level of one of ordinary skill in the art. It should be noted that no obvious morphogenic protein-induced pathological lesions arise when mature protein (e.g., OP-1, 20 mg) is administered daily to normal growing rats for 21 consecutive days. Moreover, 10 mg systemic injections of morphogen (e.g., OP-1) injected daily for 10 days into normal newborn mice does not produce any gross abnormalities.

The protein can be provided to an individual by any means suitable for systemic administration, (e.g., parenterally, as by i.v. or intraperitoneally). Liquid formulations preferably comprise part of an aqueous, physiologically acceptable solution so that in addition to delivery of the desired protein to a target site, the solution does not otherwise adversely affect the cells' or subject's electrolyte and/or volume balance. Suitable aqueous mediums include, without limitation, normal physiologic saline (e.g., 9.85% NaCl, 0.15M, pH 7-7.4). Such an aqueous solution containing the agent can be made, for example, by dissolving lyophilized protein or dispersing the protein in 50% ethanol containing acetonitrile in 0.1% trifluoroacetic acid (TFA) or 0.1% HCl, or equivalent solvents. One volume of the resultant solution then is added, for example, to ten volumes of phosphate buffered saline (PBS), which further may include 0.1-0.2%. human serum albumin (HSA). The resultant solution preferably is vortexed extensively. Alternatively, lyophilized protein can be solubilized in sodium acetate buffer (pH 4.5) or its equivalent.

Where the protein is to be provided parenterally, such as by intravenous, subcutaneous, intramuscular, intraorbital, ophthalmic, intraventricular, intracranial, intracapsular, intraspinal, intracisternal, intraperitoneal, buccal, rectal, vaginal, intranasal or by aerosol administration, the protein preferably comprises part of an aqueous solution. Currently preferred for intravenous administration is PBS or a sodium acetate buffer. The protein can be administered as a single dose or by periodic injections of a bolus of the protein, or can be made more continuous by intravenous or intraperitoneal administration from a reservoir which is external (e.g., an i.v. bag) or internal (e.g., a bioerodable implant, or a colony of implanted, morphogen-producing cells).

Useful solutions for parenteral administration may be prepared by any of the methods well known in the pharmaceutical art, described, for example, in Remington's Pharmaceutical Sciences (Gennaro, A, ed.), Mack Pub., 1990.

Another molecule capable of enhancing solubility is casein. For example, addition of 0.2% casein increases solubility of the mature active form of OP1 by 80%. Other components found in milk and/or various serum proteins also may be useful.

Morphogenic protein readily can be microencapsulated by standard phase inversion protocols. For example, morphogenic protein is added to a dilute polymer solution (i.e., 1-4% w/v in methylene chloride), which then is poured rapidly into an unstirred bath of non-solvent (petroleum ether) at a solvent to non-solvent ratio of 1:100, causing nano and microspheres (0.1-5.0 µm in diameter) to form spontaneously.

The morphogenic proteins, of course, can be administered systemically alone or in combination with other molecules known to be beneficial in the treatment of the conditions described herein. Thus, in other embodiments the present invention provides pharmaceutical compositions in which an morphogenic protein is combined with other agents which promote or enhance new skeletal tissue formation. In each such composition, the ratios or the morphogenic and mitogenic agents may be adjusted based upon their activities, as disclosed in the literature or as determined through simple experimentation, to provide a therapeutically effective dosage of each compound in a single unit dosage. The morphogenic and mitogenic agents in such a composition each preferably comprise at least about 1%, and more preferably more than 5% or 10%, of the dry weight of the composition. The compositions can, however, include other pharmaceutical carriers and active agents, as described above and, generally, in Remington's Pharmaceutical Sciences (Gennaro, A., ed.), Mack Pub., 1990, and, therefore, the morphogenic and mitogenic agents can each comprise a small fraction of the final weight of the pharmaceutical composition.

Morphogenic formulations readily can be sterilized using standard procedures prior to implantation. For example, proteins conveniently can be filter-sterilized, e.g., using a 0.22 micron filter. Alternatively, chemicals, such as ethylene oxide can be used. Carrier materials, wetting agents and/or binding agents can be sterilized by exposure to chemicals, heat, or ionizing radiation. In addition, morphogenic formulations can be terminally sterilized to a sterility assurance level of 10⁻⁶ by exposure to ionizing radiation, for example, gamma or electron beam radiation. Useful dose ranges include within the range of about 0.5-4.0 megarads, preferably 2.0-3.5 megarads. See, for example, WO 96/40297, published 19 Dec. 1996.

Practice of the invention will be still more fully understood from the following examples, which are presented herein for illustration only and should not be construed as limiting the invention-in any way.

### Example 1: Intraventricular Administration of Morphogen.

In this example, morphogen, e.g., OP-1, is administered intraventricularly, by means of canuli (e.g., 26 CTA Guide, Plastics, Inc.) implanted bilaterally into the hipporampus lateral ventricles. Canuli are implanted at least one week prior to testing or damage induction. Typically animals are divided into 4 groups: Group 1: Tissue damage induction/morphogen; Group 2: Tissue damage induction/vehicle only; Group 3: Sham tissue damage/morphogen; Group 4: Sham tissue damage/vehicle only.

Timing and dosage protocols for testing in this and other administration examples include: administering morphogen, e.g., OP-1, just prior to tissue damage, (t= minus 30',) at time of tissue damage (t=0); post tissue damage (t=30', 6 hours, 24 or 48 hours post injury).
Dosage: 0.01 - 100µg. In this example, the protocol is 5 µg/side administered biweekly for 4 weeks, starting on the day of tissue damage induction. Fluid is driven by syringe pump over a period of 1 minute.

### Example 2: Intracisternal Administration of Morphogen

Animals in the treatment group receive morphogen, e.g., OP-1, intracisternally at a dose of 1 or 10 µg/injection. Control animals receive vehicle solutions lacking OP-1 but with all other components at equivalent final concentrations. To administer the injection, the animals are anesthetized with balothane in 70% NO₂/30% O₂ and placed in stereotaxic frame. Using aseptic technique, OP-1 (1 or 10 µg/injection) or an equivalent volume of vehicle are introduced by percutaneous injection (10 µl/injection) into the cisterna magna using a Hamilton syringe fitted with a 26 gauge needle (Yamada, et al., (1991) *J Cereb. Blood Flow Metab*. 11: 472-478). Before each injection, 1-2 µl of cerebrospinal fluid (CSF) is drawn back through the Hamilton syringe to verify needle placement in the subarachnoid space. Animals are randomly assigned to either of the OP-1 treatment groups or to the vehicle treatment group. In one study, intracisternal injections (10 µg/injection OP-1 or vehicle) are made biweekly for four weeks, starting 24 hours after injury, for example after stroke induction (i.e., on post-stroke days 1, 4, 8, 11, 15, 18, 22 and 25). In a scond study, animals receive two intracisternal injections (2 X 1 µg/injection OP-1, 2 X10 µg/injection OP-1 or 2 X vehicle); the first injection is administered 24 hours after stroke and the second injection is administered 4 days after stroke. In a third study, a single injection (10 µg/injection OP-1 or vehicle) is administered 24 hours after stroke. In a fourth study, morphogen or vehicle is provided just prior to or just after stroke induction (t = 30').

### Example 3: Intravenous Administration of Morphogen.

Morphogen is prepared as described above (i.e., by dissolving in 0.9% saline, pH 7.4 optionally with 100 µg/ml BSA) so that the final concentration is 1-50 µg/ml. The morphogen is administered to rats intravenously, e.g., via tail vein at a rate of 1-50 µg/kg/hour for three hours to give a final concentration of 10-50 µg/mL. Animals are infused intravenously with this solution at a rate of 0.5mL/h for 3 h to deliver a dose of 45 µg/kg/h or 135 µg/kg for the entire infusion period. Exemplary timing protocols include 30 min before or after injury, or beginning 24 hours after injury. Control traumatized animals receive intravenous infusions of 0.9% saline with 100 µg/mL BSA or saline alone.

### Example 4: Intracisternal or CSF Administration of Morphogen Nucleic Acid

For injection into the lateral ventricle, the midsagittal scalp is incised, and a small burr hole is made in the parietal region (1.0 mm posterior and 1.5 mm lateral to the bregma) with a dental drill. A needle on a Hamilton syringe is stereotactically inserted in to the right lateral ventricle (4.0 mm in depth), and 100 µL of viral suspension (1X10¹⁰ infectious units/mL) is injected over 30 minutes. The superior plane of the parietal bone is kept horizontal during the injection. The burr hole then is covered with bone wax, and the scalp sutured. After injection of nucleic acid, the rats are housed for 1, 3, or 7 days. Alternatively, nucleic acid, e.g. "naked" DNA or viral DNA, e.g., replication-deficient adenovirus, is injected directly into cerebrospinal fluid by injecting nuclear-targeted DNA driven by a suitable promoter, e.g., the cytomegalovirus promoter, into the cisterna magna of male Sprague-Dawley or Long-Evans rats. For example, 1x10⁹ infectious units of recombinant adenovirus can be injected effectively.

### Example 5: Direct Injection of Morphogen Nucleic Acid.

Following the intraventricular administration protocol described in Example 1 above, a formulation comprising the operative naked polynucleotide as described hereinabove, dispensed in an aqueous carrier, is injected into tissue in the amounts of from 10 µl per site to about 1 ml per site. The concentration of polynucleotide in the formulation preferably is from about 0.1 µg/ml to about 20 mg/ml.

### Example 6: Administration of Morphogen via Encapsulated Cell Transplantation

Still another administration rate is implanting small clusters of cells within implantable, immune-protective capsules formed by semipermeable membranes having pores that can be suitably sized to permit passage of nutrients into the cells and secretion of morphogens out of the capsule, while restricting transit of immunoglobulins, lytic factors, and immune competent cells. Suitable cells can be any cells competent to express and secrete the morphogen of interest, including osteoblasts, renal epithelial cells, hippocampal or glial cells, and the like. Alternatively, morphogen transfected cells, such as transfected fibroblasts, CHO or COS cells can be used to advantage. Here, the gene is operatively associated with a constitutive or inducible promoter. Useful promoters include, without Stations the CMV (cytomegalovirus) and MMTV (mouse mammary tumor virus) promoters, optionally boosted by an enhancer sequence, e.g., the enhancer sequence from the Rous sarcomavirus LTR. See, for example, U.S. 5,266,683, the disclosure of which is incorporated herein by reference, for a description of recombinant morphogen DNA construction.

In the example below, hippocampal neurons are harvested and encapsulated. Cells are cultured using standard conditions, preferably in an N2 or similar medium. Cells are divided into "morphagen-treated" and "untreated" control cultures. Morphogen treated cells are exposed to 1-50 ng of morphogen, e.g., OP-1,, as described herein. Purified cells then are suspended in an alginate or other biocompatible matrix, and introduced into the lumen of a porous, hollow fiber membrane, e.g., an acrylic membrane, preferably having a molecular mass cut of about 5OkDa. Other useful matrix materials include materials referred to herein as binding agents, such as polymers and copolymers of lactic acid, butyric acid, glycolic acid, collagen, calcium carbonates and the like. The membrane then is sealed at both ends and attached to a biologically inert tether, such as a silicon tether, which facilitates handling and retrieval. See, for example, (1994) Goddard et al., Cell Transplant 3: 355, for a detailed description of a suitable device fabrication. Devices for implanting in humans typically are approximately 5 cm in length, 0.9 mm in diameter, and contain about 2 million cells. Devices for implanting in primates typically are about 12mm in length; devices for rodents are proportionally smaller.

In this example, devices are implanted about 4 weeks post injury in the animal models, using standard cranial surgical techniques. Preferably the device is implanted above the blood-brain barrier, more preferably within the hippocampal region, using a stereotoxic apparatus to guide the implant location.
Subjects are tested for cognitive function 3-4 weeks post implant. Capsules subsequently are retrieved and are used for cell viability using cell markers and antibodies to morphogen, e.g., OP-1. Devices retrieved from animals evidencing improved cognitive function following cell transplantations are anticipated to contain viable cells competent to express and secrete biologically active morphogen.

### III. MODELS OF CEREBRAL TISSUE DAMAGE: HIPPOCAMPAL DAMAGE, CELL LOSS

In the examples provided below, animals are allowed to recover for 3-4 weeks following injury before behavioral training.

### Example 7: Neurotoxic Damage

Hippocampal cell destruction in male Long-Evans or Sprague-Dawley rats (250-300g) is produced by 14 microinjections (0.04-0.08 µl) of the neurotoxin, e.g., ibotenic acid, into each hemisphere according to the methods and stereotoxic coordinates of Jarrad, LE. (1989) J. Neurosci. Method 29:251-259. Briefly, a glass micropipette glued to the end of a Hamilton syringe is wed in a microinjector fitted on a stereotoxic frame. Ibotenic acid is dissolved in PBS, pH 7.4 to a concentration of about 10 mg/ml. 0.05 µl-0.10µl injections then are made at multiple sites (e.g., 26) within the hippocampus, specifically targetting the CA1 -CA3 pyramideal cells, hilar and dentate granule cells. Subjects are allowed to recover for 3-4 weeks before training. Following behavioral testing, brains are fixed in formalin solution, frozen, sliced at 30-40 µm in the coronal plane, and stained with thionin. Brain sections from a representative animal with selective damage to the hippocampus (Ammon's hom and dentate gyrus), typically show loss of dorsal ammon's hom and only a remnant of the dentate gyrus, and cell loss in all fields of the dorsal and ventral hippocampus.

### Example 8: Transient Global Ischemia

Transient global ischemia is induced in male Long-Evans or Sprague-Dawley rats (250-300g) by creating a bilateral carotid occlusion for 15-20 minutes combined with hemorrhagic hypotension to 30 mmHg. Subjects are anesthetized with sodium pentobarbital (65 mg/kg i.p.) and treated with atropine sulphate (1.0 mg/kg i.p.) to reduce respiratory tract secretions. The common carotid arteries are isolated and loosely encircled with silk ligature. The femoral artery is cannulated with tubing connected to saline-primed reservoir via a pressure transducer. Blood pressure is recorded throughout the experiment. To induce ischemia, the mean arterial pressure is reduced to 30 mmHg by allowing the animals to hemorrhage from the arterial cannula into the saline-primed reservoir. As soon as this level of hypotension has been reached, atraumatic arterial clamps are placed on the isolated carotid arteries. After 20 min of carotid occlusion, during which time mean arterial blood pressure is maintained at 30 mmHg by withdrawing or reinfusing blood as necessary, the carotid clamps are removed, and the shed blood reinfused over approximately 10 minutes. The animal is monitored for a further 20 minutes at which point the femoral cannula is removed. Throughout the course of surgery, the subject's body temperature is monitored and maintained at 35°C. In sham-ischemia control subjects, hemorrhaging and carotid artery occlusion omitted.

### Example 9: Permanent Global Ischemia

The middle cerebral artery (MCA) occlusion model is a well accepted model of a focal ischemic episode or stroke. See, for example, Gotti, et al., (1990) Brain Res. 522: 290-307. Focal ischemia is produced by obstructing blood flow through the MCA, resulting in infarction of the brain locus supplied by this artery. The MCA model is reasonably predictive of the ability and efficacy of drugs, such as morphogen, to alter functional recovery in humans in whom central nervous system tissue has been damaged or lost due to stroke. For example, the MCA model is deemed reasonably predictive of drug efficacy to restore or delectably improve motor coordination, sensory perception, speech or any other central nervous system function naturally contributed to by tissue within the territory of the MCA.

Male Long Evans or Sprague-Dawley rats weighing 250-300 grains (Charles River Labs, Cambridge) are anesthetized with 2% halothane in 70% NO₂/30% O₂. The tail artery is cannulated in order to monitor blood gases and blood glucose. Body temperature is monitored using a rectal probe and is maintained at 37 ±0.5° C with a heating pad. The proximal right middle cerebral artery (MCA) is occluded permanently using a modification of the method of Tamura, et al. (1981), *J Cereb. Blood Flow Metab 1:* 53-60). Briefly, the proximal MCA is exposed transcranially without removing the zygomatic arch or transacting the facila nerve. The artery then is electrocoagulated using a bipolar microcoagulator from just proximal to the olfactory tract to the inferior cerebral vein, and then transacted (Bederson, *et al (1986) Stroke* 17: 472-476). Rats are observed until they regain consciousness and then returned to their home cages. An antibiotic (e.g., Cefazolin sodium) 40 mg/kg, i.p.), preferably is administered to all animals on the day before and just after stroke surgery in order to prevent infection.

### Example 10: Traumatic Brain Injury (TBI)

Traumatic Brain Injury (TBI) is conducted on 14 fasted male Long-Evans or Sprague-Dawley rats weighing between 250 and 300 g. The basic surgical preparation for the fluid-percussion brain injury also is in Dietrich et al. (1994) Acta Neuropathol. (Berl) 87: 250-258. Briefly, rats are anesthetized with 3% halothane, 30% oxygen, and a balance of nitrous oxide. Tracheal intubation is performed and rats are placed in a stereotaxic frame. A 4.8-mm craniotomy then is made overlying the right parietal cortex, 3.8 mm posterior to bregma and 2.5 mm lateral to the midline. An injury tube is placed over the exposed dura and bonded by adhesive. Dental acrylic is then poured around the injury tube and the injury tube then is plugged with a gelfoam sponge. The scalp is sutured closed and the animal returned to its home cage and allowed to recover overnight.

On the next day, fluid-percussion brain injury is produced following general procedures. See, for-example, Clifton et al.,(1991) J. Cereb. Blood Flow Metab. 11:114-121. Briefly, the fluid percussion device consists of a saline-filled Plexiglas cylinder that is fitted with a transducer house and injury screw adapted for the rat's skull. The metal screw is firmly connected to the plastic injury tube of the intubated anesthetized rat (70% nitrous oxide, 1.5% halothane, and 30% oxygen), and then injury is induced by the descent of a pendulum that strikes the piston. Typically, rats undergo mild-to-moderate head injury, ranging from 1.6 to 1.9 atm. Brain temperature is indirectly monitored with a thermistor probe inserted into the right temporalis muscle and maintained at 37-37.5°C. Rectal temperature also is measured and maintained at 37°C prior to and throughout the monitoring period.

### IV. BEHAVIORAL ASSAYS

Significant research in the area of cognitive function has been carried out to date in a variety of animal model systems, including humans, rats and monkeys. As a result of this extensive research it now is known that the hippocampus is critical to memory function. Particularly vulnerable are cells in the medial temporal lobe, particularly bilaterally in the CAl pyramidal and dentate hilar neurons of the hippocampus. Research also has shown that cell loss in the hippocampus affects both "spatial memory" or "spatial learning" (the ability to learn and remember a location using visuospatial cues), and general memory function, also referred to as "declarative memory" (the ability to learn and remember associations among items or events that can be accessed flexibly to guide actions in new situations).

A number of tests evaluating spatial and non-spatial learning and memory function in a mammal have been developed and have gained acceptance by those skilled in the art. Assays for evaluating spatial learning typically are some form of a maze and the animal must learn which location(s) provide safety, food, or some desirable objective, using visuospatial cues. Well characterized assays include the water maze, where animals, typically rats, learn to locate a submerged platform place in the center of one of four quadrants of a body of water. Another well characterized assay is the radial arm maze (RAM) where the animal, typically a rat, learns to retrieve food rewards from the ends of all, or a subset, of arms. Other mazes include the Battig or enclosed maze, T-mazes and Y-mazes. (See, for example, Helen Hodges (1996) Cognitive. Brain Res. 3:167-181).

Declarative memory in humans commonly is assessed by verbal paired associate tasks, in which individuals study arbitrarily paired words and then are tested on their ability to recall the second word when presented with the first word. Bunsey and Eichenbaum have developed and disclosed several paired associate assays for evaluating declarative memory function in rats using olfactory cues. The assays are designed to assess the flexibility of access to memories as part of the learning process (inferential teaming). Their work has shown that the memory deficit following direct hippocampal injury or injury to a immediate hippocampal pathway results in a pattern of memory impairment that parallels or mimics the memory dysfunction or amnesia observed in humans following medial temporal damage. (Ref.s 1996, 1993) Specifically, the assays evaluate the affect of tissue damage on an animal's (1) transitivity ability: the ability to judge inferentially across stimuli pairs sharing a common element, (2) symmetry: the ability to associate paired elements presented in the reverse of training order, and (3) social transmission of food preferences: the ability of an animal to change its food selection pattern based on experience with a conspecific that has recently eaten a particular food.

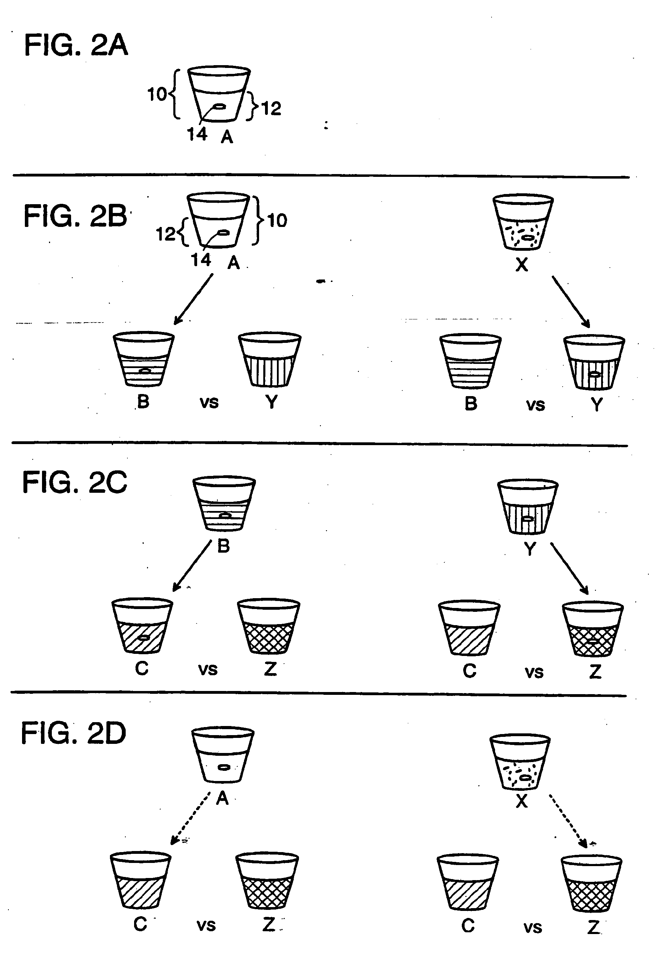

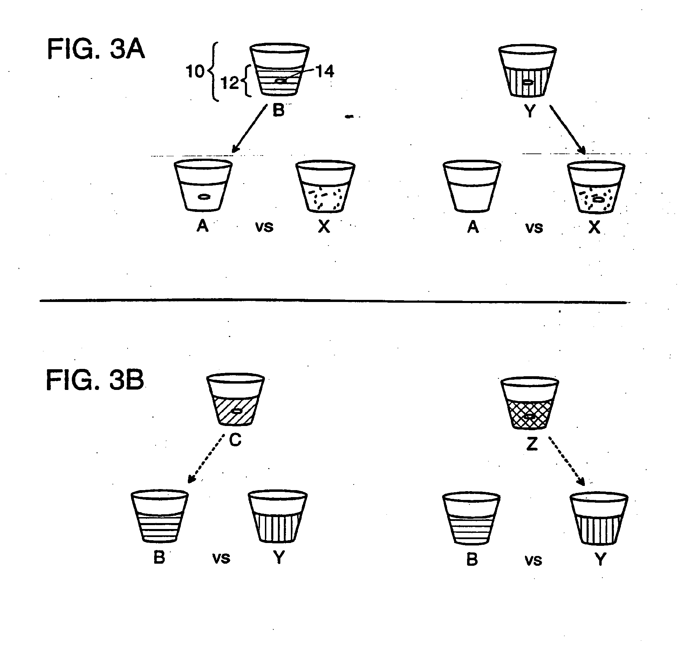

Fig. 2 is a schematic representation of the olfactory protocol generally (Fig. 2A) and the transitivity and symmetry assays (Figs. 2B-2C and Fig. 3, respectively). In the assay animals are trained on odor-odor-paired associates in two phases. In the first phase (Fig. 2A) the animal is presented with a clear cup 10 containing a mixture of sand and ground rat chow 12 which has been scented any of a variety of household spices (cup A). Buried in the mixture and not visible to the rat without digging in the mixture is a food reward 14, e.g., a Fruit Loops® or other sweet cereal. The rats are trained or "shaped" to obtain the cereal reward by digging through the mixture. In the second phase, "choice phase", the rats are presented with two choices (cups B and Y in Fig. 2B, cups C and Z in Fig. 2C), each scented with an odor different from the that of the sample cup (cup A or X in Fig. 2B, cup B or Y in Fig. 2C). In the figure, differently scented cups are indicated by different shading of the mixture 12. In the choice phase of training a reward is selectively placed in one of the choice cups, depending on the identity of the sample cup.

The transitive inference test is represented in Fig. 2D. Animals first are trained as depicted in Fig. 2A, where cup A and X are scented with cocoa and tumeric respectively, and cups B and Y are scented with coffee and salt, respectively, and are baited as depicted in the drawing. Animals then are trained on a second choice set, Fig. 2B, where cups B and Y are scented as for Fig. 2B, and cups C and Z are scented onion and nutmeg, respectively, and are baited as depicted in the drawing. On test day, subjects are presented with the choice set trials of Fig. 2B and 2C, as well as with intermixed trials, depicted in Fig. 2D. Here the choices are unbaited and the amount of time the animal spends digging in each cup for a combined maximum of 10 seconds or until digging ceases for 10 seconds is recorded. The test evaluates the ability of the animal to recall the learning of the choice sets depicted in Figs. 2B and 2C in order to make the inferential choice depicted by the hatched arrow in Fig. 2D. Normal rats, having learned the association of coca to coffee in Fig. 2B, and coffee to onion in Fig. 2C, for example, reproducibly select onion over nutmeg, when presented with cocoa as the sample cup. Rats subjected to hippocampal damage however, do not.

The symmetry test is depicted in Fig. 3. Here subjects are retrained to the criteria depicted in set 1 (Fig. 2B) except that the sample and choice cups are reversed, that is, choice cups Band Y now are sample cups, and cups A and X are choice cups. The cups are baited as indicated in the figure. The animals then are tested using the criteria in Fig. 2C, but again, with the cup order reversed. Here cups C and Z are sample cups and cups B and Y are choice cups. Choice cups are not baited and the time spent digging is recorded. Normal rats reproducibly and reliably are able to recall the learning pattern. That is, when retrained to make the coffee to cocoa selection (choice cup A) in Fig. 3A, for example, they reproducibly choose coffee (choice cup B) over salt (choice cup Y) when presented with onion (sample cup C) in Fig. 3C. Animals subjected to hippocampal damage, on the other hand, do not.

A third test assesses both immediate memory and delayed (1 day) memory for social exposure to the odor of a novel food. Research has shown that the social transmission of food preferences test, a task that involves alterations in food selection patterns consequent to experience with a conspecific that has recently eaten a particular food. When an "observer" rat encounters another ("demonstrator") rat that has recently eaten a distinctively scented food, the probability that the observer will later select that same food over other foods increases. Research also has shown that social transmission of food preferences involves the formation of a specific stimulus-stimulus association in a single training episode, plus expression of the memory in a situation different from the learning event, consistent with the general relational properties of declarative memory Cohen & Eichenbaum, (1993) Memory, Amnesia and the Hippocampal MIT Pres. Cambridge. Thus, this task is a highly "natural" form of paired associate learning, the type of task on which human amnesics typically fail to remember associations for more than a short period.

Practice of the invention will be still more fully understood from the following examples, which are presented herein for illustration only and should not be construed as limiting the invention in any way. In the Examples below, the behavioral assays typically are begun 3-4 weeks post tissue damage induction.

### Example 11: Declarative Memory - Transitive Inference Assay

The assay described herein also is provided in Bunsey et al. (1996) Nature 379: 255-257. All training and testing is performed in the home cage. Adult male Long-Evans or Sprague-Dawley rats (Charles River Labs, Cambridge, MA) first are trained or 'shaped' to obtain bits of buried sweet cereal by digging through a 50 : 50 clean sand and ground rat chow mixture contained in a 6.5 cm diameter x 6 cm tall clear plastic cup. In subsequent trainings the sample cup always has a reward buried in the cup. In the choice phase, the first cup in which a rat begins to dig is recorded as the response selection, although subjects are allowed to dig in both cups until they retrieve the reward.

Any series of household spices can be used to advantage to in this assay. In the first set of paired associates, for example, the spice odors are cocoa (cup A, 7% by weight) or 5% turmeric (Cup X) and the choice odours are 15% coffee (Cup Y) and 30% salt (Cup B). See Fig 2B. The odor pairing assignments are counterbalanced across subjects, and the left-right positioning of the choice cups is randomized. Each day multiple trials e.g., 14 trials, are conducted until subjects reach a criterion of at least 11 correct on 3 out of 4 consecutive days. To confirm that subjects are not solving the task by directly detecting buried rewards on trials when the cups are baited, for 2-3 trials per day neither choice cup is baited; instead, the reward is dropped on the surface of the sand mixture following the choice response. In the second set of paired associates sample odors are coffee (Cup B) and salt (Cup Y), the same odors as the choice items from the first set. For choice items on this set, coffee is paired with 4% onion (Cup Z), and salt is paired with 2% nutmeg (Cup C), (Fig 2C). On Test Day, subjects are presented with 10 trials from both sets of paired associates and 4 intermixed probe trials, each trial consisting of one of the samples from the first set and both choice items from the second set. The choices on these trials are unbaited and the amount of time spent digging in each of the choice cups for a combined maximum of 10 sec or until digging ceases for 10 sec is recorded.

### Example 12: Declarative Memory - Symmetry Test

Subjects are retrained to criteria on set 1 using the same protocol, but with the sample and choice items reversed in their order of presentation, i.e., sample items now are the choice items, and the original choice items now are the sample items. (See Fig 3A). Then, using the same protocol, the animals are given one 14-trial reinforced session using Set 2, with the sample and choice items reversed in order of presentation. (See Fig. 3B). On each trial the first digging response is recorded and these choices are used to calculate the preference index.

### Example 13: Declarative Memory - Social Transmission of Food Preferences

This protocol also is described in Bunsey et al. (1995) Hippocampus 5: 546-556. Subjects are initially shaped to eat ground rat chow from 6 oz cups. The day before training, initial food odor preferences are determined for chow scented with different types of ordinary spices (e.g. cinnamon, chocolate). In this preference test, the rat is presented with two weighed, differently scented food cups mounted 1 cm apart on a Plexiglas plate. Two hours later the cups are removed and weighed again, and the total amount of each diet consumed from each is recorded.

The next day rats from a pool separate from that of the subjects are used as "demonstrators" to present the odors during the training event. Demonstrator rats are food-deprived for 20 hr, and then allowed 30 min access to a cup containing ground rat chow mixed with one of the distinctive scents. Assuming at least 1 g of food is eaten by the demonstrator the food then is placed into the home cage of one of the experimental ("observer") subjects. The two rats then are allowed to interact for 20 min, after which the demonstrator is returned to its home cage. Either immediately or 24 hr later, the observer's food pellets are removed and the preference test is repeated. The ratio of the weight of the "trained" food (i.e. that eaten by the demonstrator) consumed and the total weight of food eaten is used to calculate percent trained-food preference. Preference for the "trained" food odor before and after training are compared to determine shifts in odor preference. It is anticipated that normal rats will show a strong preference for the "trained" food odor in the immediate retention test demonstrating intact odor perception as well as social and motivation mechanisms supporting this type of learned odor association. Intact rats also maintain the learned preference in the delayed retention test. However, injured rats will show a decline in delayed retention performance. Animals subjected to injury and morphogen treatment are expected to show reduced deficit on these behavioral tasks, and-to performed more like intact rats.

### Example 14: Spatial Memory - Water Maze

Cognitive deficits can be assessed using a standard water maze navigational task. (See, for example, Alexis, et al., (1995) Stroke 26:2338-2346.) Briefly, a circular tank (4 ft diameter) in a room with visual cues, is filled with water (21 ° C) and made opaque with white paint. A platform, typically 5.25 in. diameter, is hidden just beneath the water surface in one quadrant, e.g., the northeast quadrant. The animal's (typically a rat) path length and latency to find the platform are recorded, typically with an automated tracking system. The animal is trained for 2 days prior to injury, with performance assessed during two blocks of four trials each day. The animal is placed randomly at each of four starting points (north, south, east, west) and allowed 60 sec to find the hidden platform. After locating the platform, the animal is allowed to remain on the platform for 10 sec. The intertrial interval typically is approximately 4 min.

Following injury animals are given 8 standard test trials, as described above. Following the eighth trial, animals are given a "probe" trial, which consists of removing the escape platform from the water, and tracking the animal's swim pattern for 60 sec. Animals that have knowledge of the platform location spend a disproportionate amount of time in the quadrant of the maze formerly containing the platform. The probe trial is useful since it is possible for the animals, under some circumstances, to solve the task fairly efficiently without knowing the platform location. Trial timing can start 48 hours after injury and/or morphogen treatment or later, e.g. 1-3 weeks. Animals subject to hippocampal injury by any means, TBI, ischemia, etc., typically have poor recall of the platform location. It is anticipated that, following morphogen administration injured rats have reduced memory deficit and perform more like normal rats than like untreated injured rats.

### Example 15: Spatial Memory - Radial Arm Maze (RAM)

Cognitive function, particularly working memory, can be assessed using the radial arm maze. Here, animals, typically rodents, e.g., rats, learn to retrieve food rewards from the ends of all or a subset of arms that extend radially from a central platform. Typically there are eight arms. The maze can be made more complex by the addition of side arms. Working memory errors are identified when an animal re-enters an arm within a trial, and reference memory errors occur when the animal visits arms that never contain rewards. By including movable textured inserts within arms, the maze can be used to assess associative learning, as well as spatial learning. See, for example, Gionet et al., (1991) Stroke22:1040-1047 and Volpe et al.,(1989) Stroke 20:1700-1706.

In a typical example, the animal is subjected to extensive training before and/or after injury, on the order of 30-70 trials. For example, working memory following ischemic injury can be assessed by inducing the injury by a standard means, and beginning trials about four weeks post surgery. In the trials, all arms are baited and the number of within-trial reentries are measured. Alternatively, rats can be extensively pre-trained in a maze with 4/8 arms consistently baited prior to injury. Animals subjected to hippocampal injury, e.g., ischemic injury, typically have poor recall of baited arms and demonstrate a significant increase in the number of times the animal visits arms which never contain rewards, as compared with control rats. It is anticipated that, following morphogen administration, injured animals, e.g., rats, have reduced memory deficit, and perform more like normal rats than like untreated, injured rats.

### IV. BIOASSAY

### A. Bioassay of Morphogenic Activity: Endochondral Bone Formation and Related Properties

The art-recognized bioassay for bone induction as described by Sampath and Reddi, (1983) Proc. Natl. Acad. Sci. USA 80:6591-6595, and US Pat. No. 4,968,590, the disclosure of which is herein incorporated by reference, can be used to establish the efficacy of a given device or formulation. Briefly, the assay consists of depositing test samples in subcutaneous sites in recipient rats under ether anesthesia. A vertical incision (1 cm) is made under sterile conditions in the skin over the thoracic region, and a pocket is prepared by blunt dissection. In certain circumstances, approximately 25 mg of the test sample is implanted deep into the pocket and the incision is closed with a metallic skin clip. The heterotropic site allows for the study of bone induction without the possible ambiguities resulting from the use of orthotopic sites. The implants also can be provided intramuscularly which places the devices in closer contact with accessible progenitor cells. Typically intramuscular implants are made in the skeletal muscle of both legs.

The sequential cellular reactions occurring at the heterotropic site are complex. The multistep cascade of endochondral bone formation includes: binding of fibrin and fibronectin to implanted matrix, chemotaxis of cells, proliferation of fibroblasts, differentiation into chondroblasts, cartilage formation, vascular invasion, bone formation, remodeling, and bone marrow differentiation.

Successful implants exhibit a controlled progression through the stages of protein-induced endochondral bone development including: (1) transient infiltration by polymorphonuclear leukocytes on day one; (2) mesenchymal cell migration and proliferation on days two and three; (3) chondrocyte appearance on days five and six; (4) cartilage matrix formation on day seven; (5) cartilage calcification on day eight; (6) vascular invasion, appearance of osteoblasts, and formation of new bone on days nine and ten; (7) appearance of osteoblastic and bone remodeling on days twelve to eighteen; and (8) hematopoietic bone marrow differentiation in the ossicle on day twenty-one.

Histological sectioning and staining is preferred to determine the extent of osteogenesis in the implants. Staining with toluidine blue or hemotoxylin/eosin clearly demonstrates the ultimate development of endochondral bone. Twelve day bioassays are sufficient to determine whether bone inducing activity is associated with the test sample.

Additionally, alkaline phosphatase activity and/or total calcium content can be used as biochemical markers for osteogenesis. The alkaline phosphatase enzyme activity can be determined spectrophotometrically after homogenization of the excised test material. The activity peaks at 9-10 days *in vivo* and thereafter slowly declines. Samples showing no bone development by histology should have no alkaline phosphatase activity under these assay conditions. The assay is useful for quantitation and obtaining an estimate of bone formation very quickly after the test samples are removed from the rat. The results as measured by alkaline phosphatase activity level and histological evaluation can be represented as "bone forming units". One bone forming unit represents the amount of protein that is needed for half maximal bone forming activity on day 12. Additionally, dose curves can be constructed for bone inducing activity *in vivo* at each step of a purification scheme by assaying various concentrations of protein. Accordingly, the skilled artisan can construct representative dose curves using only routine experimentation.

Total calcium content can be determined after homogenization in, for example, cold 0.15M NaCl, 3 mM NaHCO₃, pH 9.0, and measuring the calcium content of the acid soluble fraction of sediment.

### B. Bioassay of Morphogenic Activity: Morphogen-Induced CAM Expression

The morphogens described herein induce CAM expression, particularly N-CAM expression, as part of their induction of morphogenesis. CAMs are morphoregulatory molecules identified in all tissues as an essential step in tissue development. N-CAMs, which comprise at least 3 isoforms (N-CAM-180, N-CAM-140 and N-CAM-120, where "180", "140" and "120" indicate the apparent molecular weights of the isoforms as measured by polyacrylamide gel electrophoresis) are expressed at least transiently in developing tissues, and permanently in never tissue. Both the N-CAM-180 and N-CAM-140 isoforms are expressed in both developing and adult tissue. The N-CAM-120 isoform is found only in adult tissue. Another neural CAM is L1.

N-CAMs are implicated in appropriate neural development, including appropriate nuerulation, neurolan migration, fasciculation, and synaptogenesis. Inhibition of N_CAM production, as by complexing the molecule with an N-CAM-specific antibody, inhibits retina organization, including retinal axon migration, and axon regeneration in the peripheral nervous system, as well as axon synapsis with target muscle cells. In addition, significant evidence indicates that physical or chemical trauma to neurons, oncogenic transformation and some genetic neurological disorders are accompanied by changes in CAM expression, which alter the adhesive or migratory behavior of these cells. Specifically, increased N-CAM levels are reported in Huntington's disease striatum (e.g., striatal basal ganglia), and decreased adhesion is noted in Alzheimer's disease.

The morphogens described herein can stimulate CAM production, particularly L1 and N-CAM production, including all three isoforms of the N-CAM molecule. For example, N-CAM expression is stimulated significantly in morphogen-treated NG108-15 cells. Untreated NG108-15 cells exhibit a fibroblastic, or minimally differentiated morphology and express only the 180 and 140 isoforms of N-CAM normally associated with a developing cell. Following morphogen treatment these cells exhibit a morphology characteristic of adult neurons and express enhanced levels of all three N-CAM isoforms. Using a similar protocol as described in the example below, morphogen treatment of NG108-15 cells also induced L1 expression.

In this example NG 108-15 cells were cultured for 4 days in the presence of increasing concentration of OP-1 and standard Wester blots performed on whole cell extracts. N-CAM isoforms were detected with an antibody which crossreacts with all three isoforms, mAb H28.123, obtained from Sigma Chemical Co., St. Louis, the different isoforms being distinguishable by their different mobilities on an electrophoresis gel. Control NG108-15 cells (untreated) express both the 140 kDa and the 180 kDa isoforms; but not the 120 kDa, as determined by western blot analyses using up to 100 µg of protein. Treatment of NG108-15 cells with OP-1 resulted in a dose-dependent increase in the expression of the 180 kDa and 140 kDa isoforms, as well as the induction of the 120 kDa isoform. The increase in N-CAM expression corresponded in a dose-dependent manner with the morphogen induction of multicellular aggregates. The induction of the 120 isoform also indicates that morphogen-induced redifferentiation of transformed cells stimulates not only redifferentiation of these cells from a transformed phenotype, but also differentiation to a phenotype corresponding to a developed cell. Standard immunolocalization studies performed with the mAb H28.123 on morphogen-treated cells show N-CAM cluster formation associated with the periphery and processes of treated cells and no reactivity with untreated cells. Moreover, morphogen treatment does not appear to inhibit cell division as determined by cell counting or ³H-tymidine uptake. Finally, known chemical differentiating agents, such as Forskolin and dimethylsulfoxide do not induce N-CAM production.

In addition, the cell aggregation effects of OP-1 on NG108-15 cells can be inhibited with anti-N-CAM antibodies or antisense N-CAM oligonucleotides. Antisense oligonucleotides can be made synthetically on a nucleotide synthesizer, using standard means known in the art. Preferably, phosphorothioate oligonucleotides ("S-oligos") are prepared, to enhance transport of the nucleotides across cell membranes. Concentration of both N-CAM antibodies and N-CAM antisense oliognucleotides sufficient to inhibit N-CAM induction also inhibited formation of multilayered cell aggregates. Specifically, incubation of morphogen-treated NG108-115 cells with 0.3-3 µM N-CAM antisense S-oligos, 5-500 µM unmodified N-CAM antisense oligos, or 10 µg/ml mAb H28.123 significantly inhibits cell aggregation. It is likely that morphogen treatment also stimulates other CAMS, as inhibition is not complete.

The experiments also have been performed with soluble morphogen (e.g., mature OP-1 associated with its pro domain) which also specifically induced CAM expression.

### C. Bioassay of Morphogenic Activity: Morphogen Induced Dendrite Outgrowth, Differentiation and Synaptogenesis

The morphogens described herein are competent to enhance the rate and degree of dendrite morphogenesis substantially. Specifically, a single application of morphogen to cultured hippocampal neurons will produce tapered and highly branched dendrites at more than twice the rate of cultured cells. In addition, morphogen also stimulates synapse formation ("synaptogenesis") in neurons. Specifically, in the presence of morphogen the number of presynaptic contacts is significantly increased in cultured neurons as compared with untreated cells.

In this example, low density hippocampal cultures were prepared as follows. Cells were dissociated by treatment with trypsin (0.25%, 15 min., 37°C), triturated, and plated at low density onto poly-L-lysine treated coverslips (1,000 cells/cm²). Once the cells became attached, at about 2 hours, the coverslips were inverted and cells were maintained in serum-free N2.1 medium, (e.g., minimum essential medium (MEM, Gibco) with the addition of standard N2 supplements, pyruvate (0.1 mM) and ovalbumin (1 mg/ml). Cells were divided into two groups: "untreated cells" - coverslips were inverted into serum-free N2.1 medium, no glial cells were present; and "morphogen-treated" cells - coverslips were treated identically to the untreated group, but 30 ng/ml OP-1 was added to the medium.

To facilitate synapse formation, a heterochronic culture technique was used (see, e.g., Fletcher et al., (1988) J. Neurosci. 14: 6695-6706.). Neurons were grown as described above for 2 days. In a separate culture procedure, new neurons were dissociated and plated on the coverslips containing the 2 day neurons. These heterochronic co-cultures were placed in dishes containing N2.1 medium and a feeder layer of glial cells. The cells were fixed 1 and 2 days later and immunostained with antibodies against synapsin 1 and MAP2, a marker of dendrite morphogenesis.

MAP2 RNA expression was measured using a cDNA MAP2 probe radiolabelled with ³⁵S-UTP using standard techniques (e.g., Sambrook et al. (1989) Molecular Cloning: A Laboratory Manual (2^{nd} ed.) Cold Spring Harbor: Cold Spring Harbor Laboratory Press). Protein was detected as follows. Cells were fixed in 4% formaldehyde in PBS (pH 7.3) containing 0.12 M sucrose at 37° for 15 min., permeabilized in 0.25% Triton X-100 for 10 min. at room temperature and rinsed in PBS. Cultures were incubated with 10% BSA at 37° for 1 hour, then in primary antibody diluted in 2% BSA in PBS at 4° overnight. Primary antibodies used were monoclonal and/or polyclonal antibodies against MAP2, β-Tubulin, Tau-1, synapsin 1, glutamate receptor. Cells were then incubated in either fluorescent-conjugated secondary antibodies (e.g., affinity-purified goat α-rabbit rhodamine, 1:400; Boehringer Mannheim, Indianapolis, IN; affinity purified goat α-mouse fluorescein, 1:400 Boehringer Mannheim, Indianapolis, IN) or biotinylated secondary antibodies (horse α-mouse IgG, 1:700; Vector Laboratories, goat α-rabbit, 1:400; Vector Laboratories) followed by the Vectastain Elite Standard ABC kit (1:50), then reacted with diaminobenzidine and H₂O₂. FM1-43 Labeling. Cells were transferred into incubation saline (119 mM NaCl, 2.5 mM KC1, 2 mM CaCl₂, 2 mM MgCl₂, 25 mM Hepes pH 7.4, and 30 mM glucose), 37° C which also contained 15 µM of the styrl dye FM-143 (Molecular Probes, Eugene, OR) and 50 mM KC 1 for 60 s and then washed in incubation saline for 5 min. Cells were viewed by microscope and images recorded using standard. Quantitative length measurements were made using Image 1 (Universal Imaging). Synaptic contact was measured by counting the number of presynaptic vesicle clusters along dendrites and the cell body. Statistical analyses were performed using General Linear Model ANOVA from the PCSAS statistics package (SAS Institute).

Addition of morphogen to cultured hippocampal neurons significantly accelerates dendritic outgrowth and development. Morphological characteristics typical of mature dendritic arbor, (such as taper and branching) are not commonly observed in these neurons until 7-14 days in vitro ("DIV"). In morphogen-treated cells these characteristics are apparent by 3DIV. In addition, morphogen-treated neurons have increased numbers of synapses as compared with untreated cells. Similarly, MAP2 expression is enhanced significantly, demonstrating the ability of morphogen to stimulate neuron, namely dendrite morphogenesis.

### Equivalents

The invention may be embodied in other specific forms without departing from the spirit or essential characteristics thereof. The foregoing embodiments are therefore to be considered in all respects illustrative rather than limiting on the invention described herein. Scope of the invention is thus indicated by the appended claims rather than by the foregoing description, and all changes which come within the meaning and range of equivalency of the claims are therefore intended to be embraced therein.

## Claims

1. Use of a morphogen comprising an amino acid sequence selected from:
a) a sequence having at least 70% amino acid sequence homology with the C-terminal seven cysteine skeleton of human OP-1, resides 330-431 of SEQ ID NO: 2;
b) a sequence having greater than 60% amino acid sequence identity with said C-terminal seven cysteine skeleton of human OP-1;
c) a sequence defined by Generic Sequence 7, SEQ ID NO: 4;
d) a sequence defined by Generic Sequence 8, SEQ ID NO: 5;
e) a sequence defined by Generic Sequence 9, SEQ ID NO: 6;
f) a sequence defined by Generic Sequence 10, SEQ ID NO: 7; and
g) a sequence defined by OPX, SEQ ID NO: 3,
in manufacture of a medicament for protecting against cognitive dysfunction or for reducing memory dysfunction in a mammal.

2. Use of a morphogen selected from OP-1, OP-2, OP-3, BMP2; BMP3; BMP4; BMP5, BMP6; BMP9; BMP-10, BMP-11, BMP-12, BMP-15, BMP-3b, DPP, Vg1, Vgr, 60A protein, GDF-1, GDF-3, GDF-5, GDF-6, GDF-7, GDF-8, GDF-9, GDF-10, and GDF-11, in manufacture of a medicament for protecting against cognitive dysfunction or for reducing memory dysfunction.

3. The use according to claim 2, wherein said dysfunction is associated with tissue damage resulting from mechanical or chemical trauma, oxygen deprivation, glucose deprivation, a neurotoxin, a neuro-degenerative disorder or dementia.

4. The use according to claim 2, wherein said dysfunction is associated with tissue damage resulting from ischemia.

5. The use according to claim 2, wherein said mammal is a human.

6. The use according to claim 5, wherein said human is at risk of or is afflicted with arterial occlusion cardiac arrest or stroke.

7. The use according to claim 2, wherein said mammal is afflicted with or is at risk of amnesia.

8. The use according to claim 2, wherein said mammal is afflicted with or is at risk of a disorder selected from Alzheimer's Disease, Pick Disease, Parkinson's Disease, Lewis-body disease, dementia, pugilista, cerebral atrophy, senility, malnutrition, glucose metabolism disorder and anorexia.

9. The use according to claim 2, wherein said medicament is for administration intraventricularly, intraveneously, or intracisternal administration.

10. The use according to claim 2, wherein said cognitive dysfunction is memory deficit, amnesia, impaired spatial/non-spatial stimulus association, senility, or dementia.

11. Use of a morphogen selected from an OP-1 polypeptide, a BMP-2 polypeptide, a BMP-5 polypeptide, a BMP-6 polypeptide, and a 60A polypeptide for the manufacture of a medicament for enhancing formation and development of dendrites and synapses in hippocampal neurons, wherein said morphogen has a conserved C-terminal seven-cysteine skeleton at least about 60% identical to residues 330-431 of human OP-1 (SEQ ID NO: 2), and wherein said morphogen induces dendrite outgrowth in said hippocampal neurons.

12. Use of a morphogen selected from an OP-1 polypeptide, a BMP-2 polypeptide, a BMP-5 polypeptide, a BMP-6 polypeptide, and a 60A polypeptide for the manufacture of a medicament for enhancing formation and development of dendrites and synapses in hippocampal neurons, wherein said morphogen has a conserved C-terminal seven-cysteine skeleton at least 70% homologous to residues 330-431 of human OP-1 (SEQ ID NO: 2), and wherein said morphogen induces dendrite outgrowth in said hippocampal neurons

13. The use of claim 11 or 12, wherein said morphogen protects against cognitive dysfunction in a mammal.

14. The use of claim 11 or 12, wherein said morphogen reduces memory dysfunction in a mammal.

15. The use of any of claims 11-14, wherein said morphogen comprises residues 30-292 of SEQ ID NO: 2.

16. The use of any one of claims 11-14, wherein said morphogen comprises residues 330-431 of SEQ ID NO: 2.

17. The use of any one of claims 11-14, wherein said morphogen comprises residues 48-292 of SEQ ID NO: 2.

18. The use of any one of claims 11-14, wherein said morphogen comprises the amino acid sequence of SEQ ID NO: 2.

19. The use of any one of claims 11-14, wherein said morphogen comprises residues 293-329 of SEQ ID NO: 2.

20. The use of any one of claims 11-14, wherein said morphogen comprises residues 293-431 of SEQ ID NO: 2.

21. The use of any one of claims 11-14, wherein said morphogen comprises residues 30-431 of SEQ ID NO: 2.


SEQUENCE LISTING

<110> Charette, Marc F
      CREATIVE BIOMOLECULES INC

<120> Methods and Compositions for Enhancing Cognitive
      Function Using Morphogenic Proteins

<130> CRP-141PC

<140>
<141>

<150> US 09/012,846
<151> 1998-01-23

<160> 9

<170> PatentIn Ver. 2.0

<210> 1
<211> 1822
<212> DNA
<213> Homo sapiens

<220>
<221> CDS
<222> (49)..(1341)

<400> 1
ggtgcgggcc cggagcccgg agcccgggta gcgcgtagag ccggcgcg atg cac gtg   57
                                                     Met His Val
                                                       1

cgc tca ctg cga gct gcg gcg ccg cac agc ttc gtg gcg ctc tgg gca   105
Arg Ser Leu Arg Ala Ala Ala Pro His Ser Phe Val Ala Leu Trp Ala
    5                  10                  15

ccc ctg ttc ctg ctg cgc tcc gcc ctg gcc gac ttc agc ctg gac aac   153
Pro Leu Phe Leu Leu Arg Ser Ala Leu Ala Asp Phe Ser Leu Asp Asn
 20                  25                  30                  35

gag gtg cac tcg agc ttc atc cac cgg cgc ctc cgc agc cag gag cgg   201
Glu Val His Ser Ser Phe Ile His Arg Arg Leu Arg Ser Gln Glu Arg
                 40                  45                  50

cgg gag atg cag cgc gag atc ctc tcc att ttg ggc ttg ccc cac cgc   249
Arg Glu Met Gln Arg Glu Ile Leu Ser Ile Leu Gly Leu Pro His Arg
             55                  60                  65

ccg cgc ccg cac ctc cag ggc aag cac aac tcg gca ccc atg ttc atg   297
Pro Arg Pro His Leu Gln Gly Lys His Asn Ser Ala Pro Met Phe Met
         70                  75                  80

ctg gac ctg tac aac gcc atg gcg gtg gag gag ggc ggc ggg ccc ggc   345
Leu Asp Leu Tyr Asn Ala Met Ala Val Glu Glu Gly Gly Gly Pro Gly
     85                  90                  95

ggc cag ggc ttc tcc tac ccc tac aag gcc gtc ttc agt acc cag ggc   393
Gly Gln Gly Phe Ser Tyr Pro Tyr Lys Ala Val Phe Ser Thr Gln Gly
100                 105                 110                 115

ccc cct ctg gcc agc ctg caa gat agc cat ttc ctc acc gac gcc gac   441
Pro Pro Leu Ala Ser Leu Gln Asp Ser His Phe Leu Thr Asp Ala Asp
                120                 125                 130

atg gtc atg agc ttc gtc aac ctc gtg gaa cat gac aag gaa ttc ttc   489
Met Val Met Ser Phe Val Asn Leu Val Glu His Asp Lys Glu Phe Phe
            135                 140                 145

cac cca cgc tac cac cat cga gag ttc cgg ttt gat ctt tcc aag atc   537
His Pro Arg Tyr His His Arg Glu Phe Arg Phe Asp Leu Ser Lys Ile
        150                 155                 160

cca gaa ggg gaa gct gtc acg gca gcc gaa ttc cgg atc tac aag gac   585
Pro Glu Gly Glu Ala Val Thr Ala Ala Glu Phe Arg Ile Tyr Lys Asp

    165                 170                 175

tac atc cgg gaa cgc ttc gac aat gag acg ttc cgg atc agc gtt tat 633
Tyr Ile Arg Glu Arg Phe Asp Asn Glu Thr Phe Arg Ile Ser Val Tyr
180                 185                 190                 195

cag gtg ctc cag gag cac ttg ggc agg gaa tcg gat ctc ttc ctg ctc 681
Gln Val Leu Gln Glu His Leu Gly Arg Glu Ser Asp Leu Phe Leu Leu
                200                 205                 210

gac agc cgt acc ctc tgg gcc tcg gag gag ggc tgg ctg gtg ttt gac 729
Asp Ser Arg Thr Leu Trp Ala Ser Glu Glu Gly Trp Leu Val Phe Asp
            215                 220                 225

atc aca gcc acc agc aac cac tgg gtg gtc aat ccg cgg cac aac ctg 777
Ile Thr Ala Thr Ser Asn His Trp Val Val Asn Pro Arg His Asn Leu
        230                 235                 240

ggc ctg cag ctc tcg gtg gag acg ctg gat ggg cag agc atc aac ccc 825
Gly Leu Gln Leu Ser Val Glu Thr Leu Asp Gly Gln Ser Ile Asn Pro
    245                 250                 255

aag ttg gcg ggc ctg att ggg cgg cac ggg ccc cag aac aag cag ccc 873
Lys Leu Ala Gly Leu Ile Gly Arg His Gly Pro Gln Asn Lys Gln Pro
260                 265                 270                 275

ttc atg gtg gct ttc ttc aag gcc acg gag gtc cac ttc cgc agc atc 921
Phe Met Val Ala Phe Phe Lys Ala Thr Glu Val His Phe Arg Ser Ile
                280                 285                 290

cgg tcc acg ggg agc aaa cag cgc agc cag aac cgc tcc aag acg ccc 969
Arg Ser Thr Gly Ser Lys Gln Arg Ser Gln Asn Arg Ser Lys Thr Pro
            295                 300                 305

aag aac cag gaa gcc ctg cgg atg gcc aac gtg gca gag aac agc agc 1017
Lys Asn Gln Glu Ala Leu Arg Met Ala Asn Val Ala Glu Asn Ser Ser
        310                 315                 320

agc gac cag agg cag gcc tgt aag aag cac gag ctg tat gtc agc ttc 1065
Ser Asp Gln Arg Gln Ala Cys Lys Lys His Glu Leu Tyr Val Ser Phe
    325                 330                 335

cga gac ctg ggc tgg cag gac tgg atc atc gcg cct gaa ggc tac gcc 1113
Arg Asp Leu Gly Trp Gln Asp Trp Ile Ile Ala Pro Glu Gly Tyr Ala
340                 345                 350                 355

gcc tac tac tgt gag ggg gag tgt gcc ttc cct ctg aac tcc tac atg 1161
Ala Tyr Tyr Cys Glu Gly Glu Cys Ala Phe Pro Leu Asn Ser Tyr Met
                360                 365                 370

aac gcc acc aac cac gcc atc gtg cag acg ctg gtc cac ttc atc aac 1209
Asn Ala Thr Asn His Ala Ile Val Gln Thr Leu Val His Phe Ile Asn
            375                 380                 385

ccg gaa acg gtg ccc aag ccc tgc tgt gcg ccc acg cag ctc aat gcc 1257
Pro Glu Thr Val Pro Lys Pro Cys Cys Ala Pro Thr Gln Leu Asn Ala
        390                 395                 400

atc tcc gtc ctc tac ttc gat gac agc tcc aac gtc atc ctg aag aaa 1305
Ile Ser Val Leu Tyr Phe Asp Asp Ser Ser Asn Val Ile Leu Lys Lys
    405                 410                 415

tac aga aac atg gtg gtc cgg gcc tgt ggc tgc cac tagctcctcc 1351
Tyr Arg Asn Met Val Val Arg Ala Cys Gly Cys His
420                 425                 430

gagaattcag accctttggg gccaagtttt tctggatcct ccattgctcg ccttggccag 1411

gaaccagcag accaactgcc ttttgtgaga ccttcccctc cctatcccca actttaaagg 1471

tgtgagagta ttaggaaaca tgagcagcat atggcttttg atcagttttt cagtggcagc 1531

atccaatgaa caagatccta caagctgtgc aggcaaaacc tagcaggaaa aaaaaacaac 1591

gcataaagaa aaatggccgg gccaggtcat tggctgggaa gtctcagcca tgcacggact 1651

cgtttccaga ggtaattatg agcgcctacc agccaggcca cccagccgtg ggaggaaggg 1711

ggcgtggcaa ggggtgggca cattggtgtc tgtgcgaaag gaaaattgac ccggaagttc 1771

ctgtaataaa tgtcacaata aaacgaatga atgaaaaaaa aaaaaaaaaa a 1822

<210> 2
<211> 431
<212> PRT
<213> Homo sapiens

<400> 2
Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Gly Gly
                85                  90                  95

Gly Pro Gly Gly Gln Gly Phe Ser Tyr Pro Tyr Lys Ala Val Phe Ser
            100                 105                 110

Thr Gln Gly Pro Pro Leu Ala Ser Leu Gln Asp Ser His Phe Leu Thr
        115                 120                 125

Asp Ala Asp Met Val Met Ser Phe Val Asn Leu Val Glu His Asp Lys
    130                 135                 140

Glu Phe Phe His Pro Arg Tyr His His Arg Glu Phe Arg Phe Asp Leu
145                 150                 155                 160

Ser Lys Ile Pro Glu Gly Glu Ala Val Thr Ala Ala Glu Phe Arg Ile
                165                 170                 175

Tyr Lys Asp Tyr Ile Arg Glu Arg Phe Asp Asn Glu Thr Phe Arg Ile
            180                 185                 190

Ser Val Tyr Gln Val Leu Gln Glu His Leu Gly Arg Glu Ser Asp Leu
        195                 200                 205

Phe Leu Leu Asp Ser Arg Thr Leu Trp Ala Ser Glu Glu Gly Trp Leu
    210                 215                 220

Val Phe Asp Ile Thr Ala Thr Ser Asn His Trp Val Val Asn Pro Arg
225                 230                 235                 240

His Asn Leu Gly Leu Gln Leu Ser Val Glu Thr Leu Asp Gly Gln Ser
                245                 250                 255

Ile Asn Pro Lys Leu Ala Gly Leu Ile Gly Arg His Gly Pro Gln Asn
            260                 265                 270

Lys Gln Pro Phe Met Val Ala Phe Phe Lys Ala Thr Glu Val His Phe
        275                 280                 285

Arg Ser Ile Arg Ser Thr Gly Ser Lys Gln Arg Ser Gln Asn Arg Ser
    290                 295                 300

Lys Thr Pro Lys Asn Gln Glu Ala Leu Arg Met Ala Asn Val Ala Glu
305                 310                 315                 320

Asn Ser Ser Ser Asp Gln Arg Gln Ala Cys Lys Lys His Glu Leu Tyr

325                    330                    335

Val Ser Phe Arg Asp Leu Gly Trp Gln Asp Trp Ile Ile Ala Pro Glu
            340                 345                 350

Gly Tyr Ala Ala Tyr Tyr Cys Glu Gly Glu Cys Ala Phe Pro Leu Asn
        355                 360                 365

Ser Tyr Met Asn Ala Thr Asn His Ala Ile Val Gln Thr Leu Val His
    370                 375                 380

Phe Ile Asn Pro Glu Thr Val Pro Lys Pro Cys Cys Ala Pro Thr Gln
385                 390                 395                 400

Leu Asn Ala Ile Ser Val Leu Tyr Phe Asp Asp Ser Ser Asn Val Ile
                405                 410                 415

Leu Lys Lys Tyr Arg Asn Met Val Val Arg Ala Cys Gly Cys His
            420                 425                 430

<210> 3
<211> 102
<212> PRT
<213> Artificial Sequence

<220>
<223> Description of Artificial Sequence: OPX

<220>
<223> Wherein each Xaa is independently selected from a
      group of one or more specified amino acids as
      defined in the specification

<400> 3
Cys Xaa Xaa His Glu Leu Tyr Val Ser Phe Xaa Asp Leu Gly Trp Xaa
  1               5                  10                  15

Asp Trp Xaa Ile Ala Pro Xaa Gly Tyr Xaa Ala Tyr Tyr Cys Glu Gly
            20                  25                  30

Glu Cys Xaa Phe Pro Leu Xaa Ser Xaa Met Asn Ala Thr Asn His Ala
        35                  40                  45

Ile Xaa Gln Xaa Leu Val His Xaa Xaa Xaa Pro Xaa Xaa Val Pro Lys
    50                  55                  60

Xaa Cys Cys Ala Pro Thr Xaa Leu Xaa Ala Xaa Ser Val Leu Tyr Xaa
 65                  70                  75                  80

Asp Xaa Ser Xaa Asn Val Ile Leu Xaa Lys Xaa Arg Asn Met Val Val
                85                  90                  95

Xaa Ala Cys Gly Cys His
            100

<210> 4
<211> 97
<212> PRT
<213> Artificial Sequence

<220>
<223> Description of Artificial Sequence: Generic-Seq-7

<220>
<223> Wherein each Xaa is independently selected from a
      group of one or more specified amino acids as
      defined in the specification

<400> 4
Leu Xaa Xaa Xaa Phe Xaa Xaa Xaa Gly Trp Xaa Xaa Xaa Xaa Xaa Xaa
  1               5                  10                  15

Pro Xaa Xaa Xaa Xaa Ala Xaa Tyr Cys Xaa Gly Xaa Cys Xaa Xaa Pro

                20                  25                  30

Xaa Xaa Xaa Xaa Xaa Xaa Xaa Xaa Asn His Ala Xaa Xaa Xaa Xaa Xaa
            35                  40                  45

Xaa Xaa Xaa Xaa Xaa Xaa Xaa Xaa Xaa Xaa Xaa Xaa Cys Cys Xaa Pro
        50                  55                  60

Xaa Xaa Xaa Xaa Xaa Xaa Xaa Xaa Leu Xaa Xaa Xaa Xaa Xaa Xaa Xaa
 65                  70                  75                  80

Val Xaa Leu Xaa Xaa Xaa Xaa Xaa Met Xaa Val Xaa Xaa Cys Xaa Cys
                    85                  90                  95

Xaa


<210> 5
<211> 102
<212> PRT
<213> Artificial Sequence

<220>
<223> Description of Artificial Sequence: Generic-Seq-8

<220>
<223> Wherein each Xaa is independently selected from a
      group of one or more specified amino acids as
      defined in the specification

<400> 5
Cys Xaa Xaa Xaa Xaa Leu Xaa Xaa Xaa Phe Xaa Xaa Xaa Gly Trp Xaa
  1               5                  10                  15

Xaa Xaa Xaa Xaa Xaa Pro Xaa Xaa Xaa Xaa Ala Xaa Tyr Cys Xaa Gly
                20                  25                  30

Xaa Cys Xaa Xaa Pro Xaa Xaa Xaa Xaa Xaa Xaa Xaa Xaa Asn His Ala
            35                  40                  45

Xaa Xaa Xaa Xaa Xaa Xaa Xaa Xaa Xaa Xaa Xaa Xaa Xaa Xaa Xaa Xaa
        50                  55                  60

Xaa Cys Cys Xaa Pro Xaa Xaa Xaa Xaa Xaa Xaa Xaa Xaa Leu Xaa Xaa
 65                  70                  75                  80

Xaa Xaa Xaa Xaa Xaa Val Xaa Leu Xaa Xaa Xaa Xaa Xaa Met Xaa Val
                    85                  90                  95

Xaa Xaa Cys Xaa Cys Xaa
                100


<210> 6
<211> 97
<212> PRT
<213> Artificial Sequence

<220>
<223> Description of Artificial Sequence: Generic-Seq-9

<220>
<223> Wherein each Xaa is independently selected from a
      group of one or more specified amino acids as
      defined in the specification

<400> 6
Xaa Xaa Xaa Xaa Xaa Xaa Xaa Xaa Xaa Xaa Xaa Xaa Xaa Xaa Xaa Xaa
  1               5                  10                  15

Pro Xaa Xaa Xaa Xaa Xaa Xaa Xaa Cys Xaa Gly Xaa Cys Xaa Xaa Xaa
                20                  25                  30

Xaa Xaa Xaa Xaa Xaa Xaa Xaa Xaa Xaa Xaa Xaa Xaa Xaa Xaa Xaa Xaa

35                  40                  45

Xaa Xaa Xaa Xaa Xaa Xaa Xaa Xaa Xaa Xaa Xaa Xaa Xaa Cys Xaa Pro
    50                  55                  60

Xaa Xaa Xaa Xaa Xaa Xaa Xaa Xaa Leu Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Cys Xaa Cys
                85                  90                  95

Xaa


<210> 7
<211> 102
<212> PRT
<213> Artificial Sequence

<220>
<223> Description of Artificial Sequence: Generic-Seq-10

<220>
<223> Wherein each Xaa is independently selected from a
      group of one or more specified amino acids as
      defined in the specification

<400> 7
Cys Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Pro Xaa Xaa Xaa Xaa Xaa Xaa Xaa Cys Xaa Gly
            20                  25                  30

Xaa Cys Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Cys Xaa Pro Xaa Xaa Xaa Xaa Xaa Xaa Xaa Xaa Leu Xaa Xaa
65                  70                  75                  80

Xaa Xaa Xaa Xaa Xaa Xaa Xaa Xaa Xaa Xaa Xaa Xaa Xaa Xaa Xaa Xaa
                85                  90                  95

Xaa Xaa Cys Xaa Cys Xaa
            100


<210> 8
<211> 5
<212> PRT
<213> Artificial Sequence

<220>
<223> Wherein each Xaa is independently selected from a
      group of one or more specified amino acids as
      defined in the specification

<220>
<223> Description of Artificial Sequence: CXXXX

<400> 8
Cys Xaa Xaa Xaa Xaa
1               5


<210> 9
<211> 5
<212> PRT
<213> Artificial Sequence

<220>

<223> Description of Artificial Sequence: CXXXX

<220>
<223> Wherein each Xaa is independently selected from a
      group of one or more specified amino acids as
      defined in the specification

<400> 9
Cys Xaa Xaa Xaa Xaa
 1               5